# EUROPEAN PATENT SPECIFICATION

(11) **EP 0 410 953 B1**
(45) Date of publication and mention of the grant of the patent: **08.11.1995**
(21) Application number: 90870119.6
(22) Date of filing: 23.07.1990
(51) Int. Cl.: C07D 211/42, C07D 211/46, C07D 491/056, C07F 7/22, A61K 31/445

(54) **Piperidine compounds with glycosidase inhibition activity**
Glykosidasehemmende Piperidinverbindungen
Composés de pipéridine inhibiteurs de glycosidase

(30) Priority: 27.07.1989 US 386538; 29.08.1989 US 400252
(43) Date of publication of application: 30.01.1991
(73) Proprietor: MONSANTO COMPANY, St. Louis, Missouri 63167 (US)
(72) Inventor: Getman, Daniel Paul, St Louis, Missouri 63146 (US); De Crescenzo, Gary Anthony, St Peters, Missouri 63376 (US)
(74) Representative: Ernst, Hubert

(56) References cited:
- EP-A- 0 240 868
- EP-A- 0 298 350
- JOURNAL OF THE AMERICAN CHEMICAL SOCIETY, vol. 109, no. 24, 25 November 1987,Columbus, Ohio, pages 7530 - 7531; Stephen G.Withers et al.: "2-Deoxy-2-fluoroglucosides:A novel Class of Mechanism-Based Glucosidase Inhibitors"
- ANGEWANDTE CHEMIE, vol. 93, no. 9, 1981, Weinheim, pages 738 - 755; ErnstTruscheit et al.: "Chemie und Biochemie mikrobieller alpha-Glucosidasen-Inhibitoren"
- JOURNAL OF THE AMERICAN CHEMICAL SOCIETY, vol. 110, no. 1, 6 January 1988, Columbus, Ohio, pages 312 - 313; Michael K.Tong et al.: "A Potent New Class of Active-Site-Directed-Glycosidase Inactivators"
- TETRAHEDRON, vol. 41, no. 4, 1985, Great Britain, pages 643 - 663; S.David et al.: "Regioselective manipulation of hydroxyl groups via organotin derivatives"

## Description

### 1. Field of the Invention

This invention relates to novel piperidine derivatives which manifest glycosidase inhibition activity and to novel intermediates useful in the manufacture thereof. The present invention also relates to methods for preparing such derivatives and intermediates.

More particularly, the present invention relates to 5-fluoro analogs of 2-hydroxymethyl-3, 4,-dihydroxypiperidines and to 4-fluoro analogs of 2-hydroxymethyl-3, 5,-dihydroxypiperidines, which are the ring nitrogen analogs of 1-deoxy-D-glucose and are generally referred to as 1-deoxynojirimycin (DNJ) analogs. More particularly, the present invention relates to 1,2 dideoxy-2-fluoronojirimycin and to 1,3-dideoxy-3-fluoronojirimycin and the corresponding N-derivatives; to intermediates useful in preparing such fluorinated analogs; to methods for preparing the intermediates beginning with 1-deoxynojirimycin as starting material; and to methods for preparing the 2-fluoro and 3-fluoro analogs utilizing such intermediates.

### 2. Related Art

1-Deoxynojirimycin is a known glucosidase inhibitor. See, for example, Truscheit et al., Ang. Chemie Int'l. Ed., 20, 744 (1981). Fluoro analogs of glucose and glucose derivatives are also known. For example, see Withers et al, J.Amer. Chem. Soc., 109, 7530-31 (1987), and "Fluorinated Carbohydrates: Chemical and Biochemical Aspects; ACS Symposium Series 184," ed. N.F. Taylor, American Chemical Society (1988).

Kinast et al, DE3620645, disclose 2-amino-1-deoxynojirimycin derivatives which inhibit glucosidases. A cyclic stannylene intermediate of 1-deoxymannojirimycin is utilized to specifically functionalize the 3-hydroxy group.

Munava et al, J. Org. Chem., 41, 1832 (1976), disclose a cyclic stannylene intermediate of glucose utilized to functionalize the 2-hydroxy group with a benzoyl group.

David et al, Tetrahedron, 41(4), 643 (1985) review utilization of stannylenes in carbohydrate chemistry.

EP 0 298 350 discloses chiral 6-hydroxymethylene-3-amino-4,5-dihydroxypiperidine derivatives, their synthesis and the use of said derivatives as a medicament, particularly, for influencing the metabolism of lipids and carbohydrates. EP 0 298 350 discloses, inter alia, the use of N-derivatized 4,6-O-protected 1,5-dideoxy-1,5-imino-glucitol as an intermediate to the synthesis of 2-amino-derivatized 1,5-dideoxy-1,5-imino sugars.

### SUMMARY OF THE INVENTION

The present invention is directed at 1,2-dideoxy-2-fluoronojirimycin and 1,3-dideoxy-3-fluoronojirimyin, and the N-derivatives thereof. These compounds are prepared utilizing novel N-substituted-4,6-0-benzylidene-1,2-dideoxy-2-fluoronojirimycin intermediates, N-substituted-2,3-anhydro-4,6-0-benzylidene-1-deoxymannojirimycin intermediates, N-substituted-4,6-0-benzylidene-2-0-(sulfonyl ester)-1-deoxynojirimycin intermediates, N-substituted-4,6-0-benzylidene-1,3-dideoxy-3-fluoronojirimycin intermediates, N-substituted-4,6-0-benzylidene-2-0-substituted-1-deoxynojirimycin intermediates, N-substituted-2-0-substituted-4,6-0-benzylidene-3-keto-1-deoxynojirimycin, N-substituted-2-0-substituted-4, 6-0-protected-1-deoxyallojirimycin, and N-substituted-2,3-0-(dialkylstannylene)-4,6-0-benzylidene-1-deoxynojirimycin intermediates which are then utilized to produce the subject compounds.

### DETAILED DESCRIPTION OF THE INVENTION

The present invention resides in the discovery that novel 2-deoxy-2-fluoro and 3-deoxy-3-fluoro analogs of 1-deoxynojirimycin and the N-derivatives thereof manifest glycosidase inhibition activity. The subject compounds can be represented by the formulas: 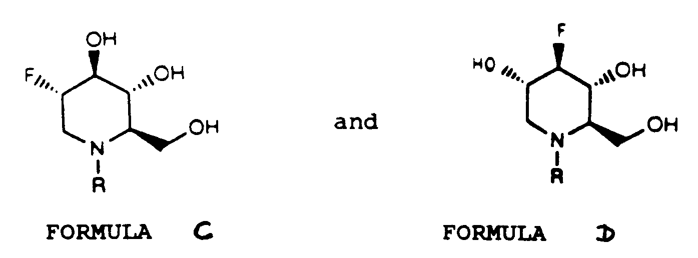
wherein R represents hydrogen, alkyl radicals having from 1 to 10 carbon atoms, alkenyl radicals having up to 10 carbon atoms, aryl, alkaryl and aralkyl radicals having from 6 to 16 carbon atoms, and acyl and acyloxy radicals of the formula 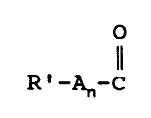
wherein R' represents alkyl radicals having from 1 to 10 carbon atoms, and aryl, aralkyl and alkaryl radicals having from 6 to 26 carbon atoms; A represents oxygen; n is 0 or 1.

Accordingly, the present invention is directed to such novel analogs, to novel intermediates useful in the manufacture of such analogs, and to methods for preparing such novel intermediates and analogs.

These novel analogs and intermediates can be represented generically by the formulas: 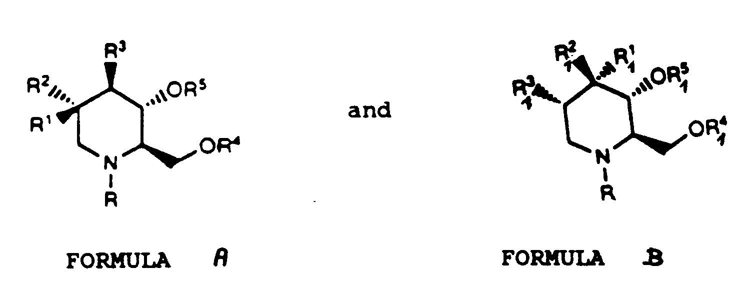
wherein R represents hydrogen, alkyl radicals having from 1 to 10 carbon atoms, alkenyl radicals having up to 10 carbon atoms, aryl, alkaryl and aralkyl radicals having from 6 to 16 carbon atoms and acyl and acyloxy radicals of the formula 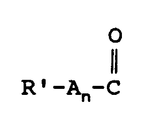
wherein R' represents alkyl radicals having from 1 to 10 carbon atoms, and aryl, aralkyl and alkaryl radicals having from 6 to 26 carbon atoms; A represents oxygen; n is 0 or 1;
R₁ represents hydrogen; R₂ represents hydrogen, fluorine, and sulfonyl esters represented by the following formula: 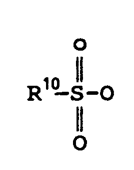
wherein R¹⁰ represents alkyl radicals having from 1 to 6 carbon atoms and aryl, aralkyl and alkaryl radicals; R³ represents hydroxy or together with R² represents a cyclic stannylene derivative of the formula: 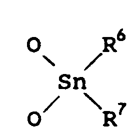
wherein R⁶ and R⁷ independently represent alkyl radicals having from 1 to 10 carbon atoms, or together with R¹ represents an epoxide; provided that when R² is fluorine, R³ is hydroxy; and when R² is hydrogen, R¹ and R³ together form an epoxide; R⁴ and R⁵ represent hydrogen and hydroxy protecting groups; R₁¹ represents hydrogen and a hydroxyl group; R₁² represents hydrogen, hydroxy and fluorine or together with R₁¹ represent keto group; R₁³ represents hydroxy, substituted and unsubstituted benzyl and allyl ethers, and acyl esters represented by the following formula: 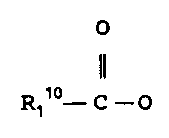
wherein R₁¹⁰ represents alkyl radicals having from 1 to 10 carbon atoms and aryl, aralkyl and alkaryl radicals or together with R₁² represents a cyclic stannylene derivative of the formula: 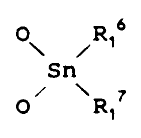
wherein R₁⁶ and R₁⁷ independently represent alkyl radicals having from 1 to 10 carbon atoms; provided that when R₁² is fluorine, R₁³ is hydroxy, benzyl or allyl ether, or acyl ester; and
when R₁¹ is hydroxy, R₁³ is hydroxy, an acyl ester or a benzyl or allyl ether, further provided that when R₁² is hydroxy, then R₁³ is not hydroxy, further provided that when R₁¹ is hydroxy, R₁² is hydrogen and further provided that only one of R₁¹ and R₁² may concurrently be hydrogen; and R₁⁴ and R₁⁵ represent hydrogen and hydroxy protecting groups.

More particularly, the novel analogs can be represented by the formulas: 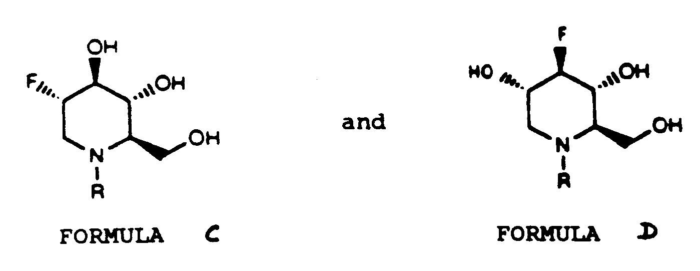
wherein R represents hydrogen, alkyl radicals having from 1 to 10 carbon atoms, alkenyl radicals having up to 10 carbon atoms, aryl, alkaryl and aralkyl radicals having from 6 to 16 carbon atoms and acyl and acyloxy radicals of the formula 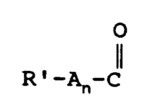
wherein R' represents alkyl radicals having from 1 to 10 carbon atoms, and aryl, aralkyl and alkaryl radicals having from 6 to 26 carbon atoms; A represents oxygen; n is 0 or 1.

The N-substituted-4,6-0-protected-1, 2-dideoxy-2-fluoronojirimycin and 3-dideoxy-3-fluoronojirimycin intermediates can be represented by the formulas: 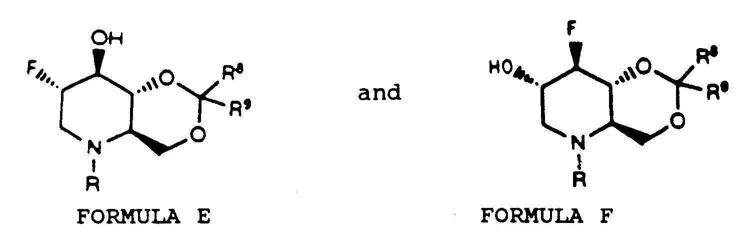
wherein R has the same meaning as set forth above and R₈ and R₉ independently represent hydrogen, alkyl radicals having from 1 to 10 carbon atoms and aryl radicals.

The epoxide intermediates of Formula E can be represented by the formula: 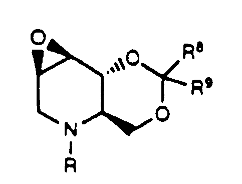
wherein R, R⁸ and R⁹ have the same meaning as set forth above.

The N-substituted-2-0-substituted-1,3-dideoxy-3-fluoronojirimycin of Formula F can be represented by the formula: 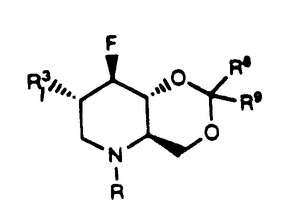
wherein R, R₁³, R⁸ and R⁹ have the same meaning as set forth above.

The N-substituted-2-0-substituted-4,6-0-protected-1-deoxyallojirimycin can be represented by the formula: 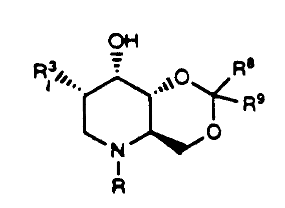
wherein R, R₁³, R⁸ and R⁹ have the same meaning as set forth above.

The N-substituted-2-0-substituted-4,6-0-benzylidene-3-keto-1-deoxynojirimycin can be represented by the formula: 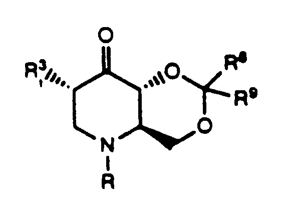
wherein R, R₁³, R⁸ and R⁹ have the same meaning as set forth above.

The 2- and 2-0-substituted intermediates can be represented by the formulas: 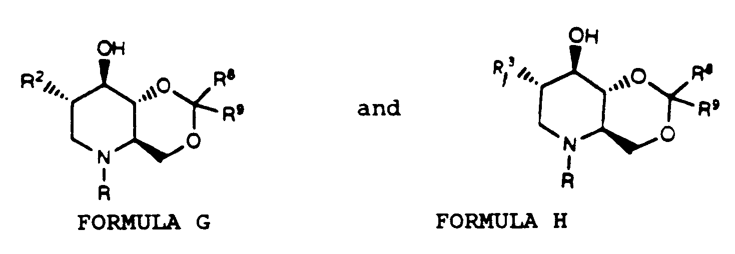
wherein R has the same meaning as set forth above; R² represents sulfonyl ester represented by the formula: 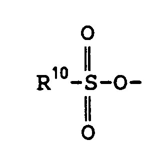
wherein R¹⁰ represents alkyl radicals having from 1 to 6 carbon atoms and aryl, aralkyl and alkaryl radicals; R₁³ represents benzyl and allyl ethers, acyl esters represented by the formula: 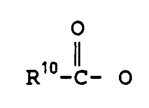
wherein R¹⁰ represents alkyl radicals having from 1 to 10 carbon atoms and aryl, aralkyl and alkaryl radicals; and R⁸ and R⁹ have the same meaning as set forth above.

The cyclic stannylene intermediates can be represented by the formula: 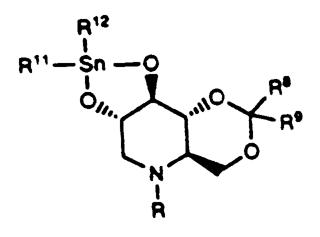
wherein R, R⁸ and R⁹ represent radicals as defined above; and R¹¹ and R¹² represent alkyl radicals having from 1 to about 10 carbon atoms.

The 1,2-dideoxy-2-fluoronojirimycin and 1,3-dideoxy-3-fluoronojirimycin compounds of the present invention can be prepared beginning with 1-deoxynojirimycin (hereinafter referred to as "DNJ"), which can be prepared by known procedures as disclosed in U.S. Patent Nos. 4,220,782; 4,246,345; and 4,806,650. The corresponding N-alkyl derivatives can then be prepared according to known procedures. See, for example, U.S. Patent Nos. 4,220,782; 4,266,025; 4,405,714; and 4,806,650.

Starting with DNJ, the N-alkyl group can first be introduced according to known procedures. The 4-hydroxy and 6-hydroxy groups are then protected by techniques well known to those familiar with carbohydrate chemistry. These N-alkyl-4,6-O-protected derivatives can be represented by the formula: 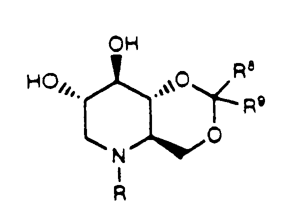
wherein R⁸ and R⁹ independently represent radicals as defined above. For example, utilizing 2,2-dimethoxypropane or, preferably, benzaldehyde, the corresponding 4,6-0-isopropylidene- (R⁸=R⁹=CH₃) or 4,6-O-benzylidene (R⁸=phenyl, R⁹=H) N-substituted DNJ can be produced. These reactions are generally conducted in an inert organic solvent and in the presence of a strong acid which acts as catalyst. The reactions can be conducted at temperatures of from about 0°C to about 50°C, preferably from about 10°C to 40°C, such as from about 20°C to about 30°C. Exemplary acid catalysts include zinc chloride and p-toluenesulfonic acid. During the reaction water is removed, preferably utilizing a molecular sieve such as a 3 angstrom (Å) molecular sieve.

Alternatively, starting with DNJ, the amino group can be protected and then the 4-hydroxy and 6-hydroxy groups are protected according to the above procedure. Protection of the amino group can be accomplished by methods well known to those familiar with amino acid chemistry. For example, the amino group can be protected utilizing a carbonyl compound represented by the formula: 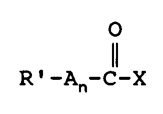
wherein R' represents alkyl radicals having from 1 to 10 carbon atoms, and aryl, aralkyl and alkaryl radicals having from 6 to 26 carbon atoms, A represents oxygen; n is 0 or 1; and X represents Cl, Br, I, or C(O)AₙR' wherein R', A and n have the same meanings as defined above. Exemplary amino protecting groups include carbobenzoxy, butyryl, benzoyl, and the like. These reactions ace generally conducted in a polar solvent and in the presence of a base at a temperature of from about 0°C to about 50°C, preferably from about 0°C to about 25°C such as from about 10°C to 20°C. Exemplary bases include NaHCO₃, NaOH and certain tertiary amines. Exemplary solvents include water and N,N-dimethylformamide.

It is preferred, however, that the compounds of the present invention be prepared starting with DNJ, protecting the amino group with the carbobenzoxy group and then protecting the 4-hydroxy and 6-hydroxy groups utilizing the benzylidene protecting group. Optionally, the amino protecting group can then be removed by procedures well known in the art, such as with a base, e.g., KOH, NaOH, and LiOH. In this case, an alkyl acid chloride is then reacted with the 4,6-O-benzylidene-1-deoxynojirimycin to produce the N-carboalkyl-4,6-O-benzylidene-1-deoxynojirimycin, the N-carboalkyl being reduced to the desired alkyl group in a subsequent step as discussed below. Alternately, the amino protecting group can be removed in a later step as discussed below.

In order to facilitate discussion of the remaining method steps, the N-alkyl, N-carboaryloxy, N-carboallyloxy, and N-carboalkyl DNJ derivatives will be collectively referred to as N-protected. Also, the 4,6-O-benzylidene compounds will be referred to as 4,6-O-protected compounds.

The above-described N-protected-4,6-O-protected-1-deoxynojirimycin is then reacted with a dialkyltin oxide (R¹¹R¹²SnO), preferably di-n-butyltin oxide, in a suitable solvent such as methanol, benzene or toluene to form the novel corresponding cyclic stannylene derivative represented by the formula: 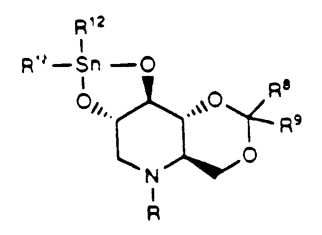
wherein R, R⁸ and R⁹ have the same meaning as set forth above and R¹¹ and R¹² independently represent alkyl radicals having from 1 to 10 carbon atoms, such as from 1 to 6 carbon atoms, preferably about 4 carbon atoms.

The cyclic stannylene derivative for formula G is then reacted with an electrophilic sulfonyl compound. Suitable electrophiles are sulfonyl halides and anhydrides. Preferred electrophiles are those represented by the formula: 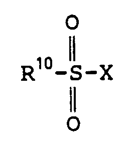
wherein X represents Cl, Br, I,
and 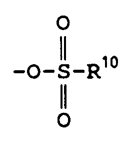
and R¹⁰ represents alkyl radicals having from 1 to 6 carbon atoms and aralkyl and aryl radicals. Exemplary R¹⁰ radicals include phenyl, p-methylphenyl, trifluoromethyl (CF₃) and methyl. A preferred electrophile is the p-toluenesulfonyl chloride. The reactions are conducted in an inert solvent in the presence of a base and at a temperature of between about 0°C and 100°C, preferably at a temperature of from about 0°C to about 25°C. Exemplary bases include tertiary amines such as triethylamine, and diisopropylethyl amine, pyridine, N,N-dimethylaminopyridine, 1,8-diazabicyclo[5.4.0]undec-7-ene (DBU) and 1,5-diazabicyclo[4.3.0]non-5-ene (DBN).

The resulting novel product of the reaction between the cyclic stannylene and the electrophile is predominantly the N-protected-2-O-substituted-4,6-O-benzylidene-1-deoxynojirimycin represented by the formula: 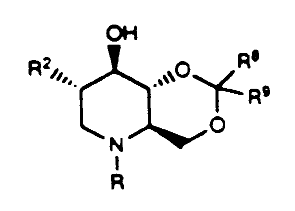
wherein R² represents a sulfonyl ester and R, R⁸ and R⁹ are as defined above. By predominant it is meant that the 2-O-substituted product is produced in excess of the 3-O-substituted product.

The sulfonyl ester at C-2 of the N-protected-2-O-substituted-4,6-O-benzylidene-1-deoxynojirimycin is then displaced under conditions which produce the corresponding N-protected-2,3-anhydro-4,6-O-benzylidene-1-deoxymannojirimycin represented by the formula: 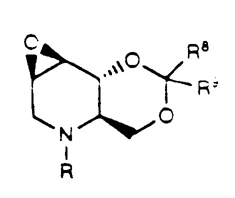
wherein R, R⁸ and R⁹ are as defined above. For example, utilizing a strong base such as sodium hydride, potassium hydride, a potassium or sodium alkoxide, e.g., sodium methoxide, sodium ethoxide, potassium t-butoxide and the like, or DBU, the sulfonyl ester at the 2-position is displaced, along with the oxygen, with inversion of configuration at C-2 to produce the corresponding 2,3-anhydro-1-deoxymannojirimycin. A preferred base is sodium hydride. The reaction is generally conducted in a suitable solvent in the presence of the base and at a temperature of between about 0°C and about 120°C, preferably at a temperature of between about 0°C and 25°C. Exemplary suitable solvents include tetrahydrofuran, methylene chloride, methanol and ethanol. A preferred solvent is tetrahydrofuran.

The N-protected-2,3-anhydro-4,6-O-benzylidene-1-deoxymannojirimycin is then reacted with a fluorine source with inversion of configuration at C-2 to produce the corresponding N-protected-4,6-O-benzylidene-1,2-dideoxy-2-fluoronojirimycin represented by the formula: 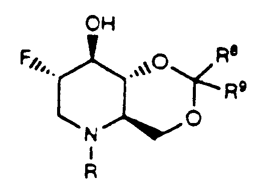
wherein R, R⁸ and R⁹ are as defined above. The reaction is preferably conducted neat, i.e., no solvent, at a temperature of between about 50°C and 150°C, preferably between about 100°C and about 140°C. Exemplary fluorine sources include those represented by the formula: 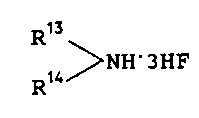
wherein R¹³ and R¹⁴ independently represent optionally substituted alkyl groups having from 1 to 6 carbon atoms. A preferred alkyl group is isopropyl. Other fluoride sources include potassium hydrogen fluoride, hydrogen fluoride, hydrogen tetrafluoroborate and tetra-alkylammonium fluorides (e.g. tetra-N-butylammonium fluoride).

The cyclic stannylene derivative for Formula H is then reacted with a suitable agent for providing the desired acyl ester, benzyl or allyl ether derivatives. These derivatives are referred to herein as 2-O-substituted derivatives. Suitable acylating agents include those represented by the formula: 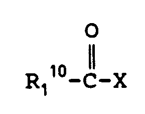
wherein X represents Cl, Br, I,
and 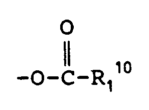
and R₁¹⁰ represents alkyl radicals having from 1 to 10 carbon atoms and aralkyl and aryl radicals. Exemplary R¹⁰ radicals include phenyl, p-methylphenyl, chloromethyl and methyl. A preferred acylating agent is benzoyl chloride. Exemplary bases include tertiary amines such as triethylamine, and diisopropylethyl amine, pyridine, N,N-dimethylaminopyridine, 1,8-diazabicyclo[5.4.0] undec-7-ene (DBU) and 1,5-diazabicyclo[4.3.0]non-5-ene (DBN). Suitable benzylating agents include benzyl triflate and benzyl halides, e.g., benzyl chloride, bromide and iodide. Suitable allylating agents include allyl halides such as allyl bromide and allyl iodide. The benzylation and allylation reactions are conducted in the presence of a catalytic amount of a tetraalkylammonium iodide, for example, tetra-n-butyl ammonium iodide, in a suitable solvent such as THF, acetonitrile or N,N-dimethylformamide. The reactions are conducted in an inert solvent in the presence of a base and at a temperature of between about 0°C and 100°C, preferably at a temperature of from about 0°C to about 25°C.

The resulting novel product of the reaction between the cyclic stannylene and the acylating, benzylatin or allylating agent is predominantly the N-protected-2-O-substituted-4,6-0-benzylidene-1-deoxynojirimycin represented by the formula: 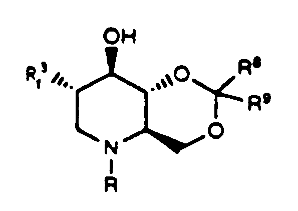
wherein R₁³ represents an acyl ester as defined above and R, R⁸ and R⁹ are as defined above. By predominant it is meant that the 2-O-substituted product is produced in excess of the 3-O-substituted product.

The N-protected-2-O-substituted-4,6-0-benzylidene-1-deoxynojirimycin is then oxidized and reduced under conditions which produce the corresponding N-protected-2-O-substituted-4,6-O-benzylidene-1-deoxyallojirimycin represented by the formula: 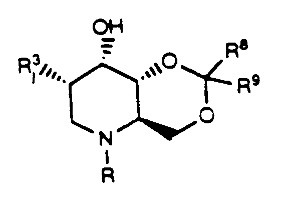
wherein R, R₁³, R⁸ and R⁹ are as defined above.

For example, oxidation an be effected utilizing a variety of oxidizing agents, for example, pyridinium chlorochromate, pyridinium dichromate and the like. Preferred oxidizing agents are those referred to as Swern reagents by those skilled in the art. Swern reagents, generally are combinations of dimethyl sulfoxide and either trifluoroacetic anhydride or oxallyl chloride, and triethylamine. The reaction is conducted in an inert solvent at a temperature of from about -80°C to about 30°C. A preferred inert solvent is methylene chloride. For a review of these Swern reagents, see A. J. Mancuso and D. Swern, Synthsis, 165 (1981) which is herein incorporated by reference. A preferred inert solvent is methylene chloride.

The resulting ketone, represented by the formula: 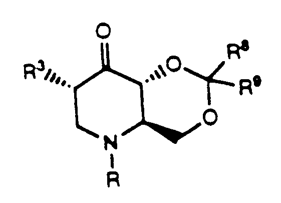
where R, R³, R⁸ and R⁹ are as defined above, is then reduced utilizing a metal borohydride such as sodium, lithium and potassium borohydride, in the presence of THF and methanol or with an alumino hydride, such as lithium trialkylaluminohydride and lithium trialkoxyaluminohydride, in THF, and at a temperature of from about -80°C to about 30°C. The resulting reduced product is the N-protected-2-0-substituted-4,6-o-benzylidene-1-deoxyallojirimycin described above.

The N-protected-2-0-substituted-4,6-O-benzylidene-1-deoxyallojirimycin is then reacted with a suitable fluorine source with inversion of configuration at C-3 to produce the corresponding N-protected-2-O-substituted-4,6-O-benzylidene-1,3-dideoxy-3-fluoronojirimycin represented by the formula: 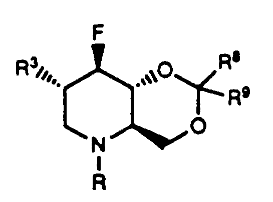
wherein R, R³, R⁸ and R⁹ are as defined above. The reaction is preferably conduced in an inert-solvent, e.g., methylene chloride, benzene, toluene, chloroform, THF, and the like, at a temperature of between about -80°C and about 120°C, preferably between about 0°C and about 85°C. Exemplary fluorine sources include those represented by the formula: 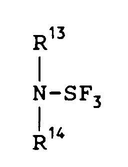
wherein R¹³ and R¹⁴ independently represent alkyl groups having from 1 to 6 carbon atoms. A preferred alkyl group is ethyl.

Alternatively, the inverted alcohol can first be activated by conversion to its trifluoromethanesulfonate or p-methylbenzenesulfonate derivative and then displaced by a fluoride source in a suitable solvent. Suitable fluoride sources include cesium fluoride, potassium hydrogen fluoride, tetraalkylammonium fluorides, e.g., tetra-n-butylammonium fluoride, and tris(dimethylamino)sulfur (trimethylsilyl)difluoride. Suitable solvents include acetonitrile and N,N-dimethylformamide. This reaction is conducted at a temperature of between about -80°C and 120°C.

The next step involves removal of the acyl, benzyl or allyl group at C-2. These groups can be removed in a variety of ways well known to those skilled in the art. For example, the acyl groups can be removed utilizing lithium hydroxide, sodium hydroxide or potassium hydroxide in aqueous tetrahydrofuran. A preferred method for removal utilizes sodium methoxide in methanol.

The next step involves removal of the 4,6-O-protecting group, e.g., the benzylidene group, by methods well known to those skilled in the art. Generally, such protecting groups can be removed utilizing an acid in an appropriate solvent at room temperatures. For example, CF₃CO₂H in water, CH₃CO₂H in water or HCl in water can be utilized to effectively deprotect the 4- and 6-hydroxy groups. Alternatively, such protecting groups can be removed catalytically. For example, reaction with palladium on carbon at 50°C and 3.5 Kg/sq.cm. H₂.

It should be noted that where the amino protecting group can be removed through hydrogenolysis (for example, a carbobenzoxy group), hydrogenation in the presence of palladium on carbon will remove both the nitrogen protecting group and the 4,6-O-benzylidene protecting group. Thus, deprotection can occur in one step.

Alternatively, the acyl group can be reduced utilizing borane:dimethylsulfide, lithium aluminum hydride or diborane in a suitable solvent, e.g., THF, at a temperature of from about 0°C to about 120°C, preferably from about 0°C to about 25°C. A preferred reducing agent is borane:dimethylsulfide Following reduction of the acyl group to the corresponding alkyl group, the 4- and 6-hydroxy protecting groups can be removed as described above.

Where other amino protecting groups are utilized, however, one can remove such groups either prior to or following deprotection of the 4-and 6-hydroxy groups utilizing well known methods and, if desired, replace such groups with an appropriate alkyl group by methods well known in the art.

The subject 1,2-dideoxy-2-fluoronojirimycin, 1,3-dideoxy-3-fluoronojirimycin and N-derivatives thereof manifest glycosidase inhibition activity. It is contemplated that certain intermediates disclosed herein will manifest similar activity. Thus, pharmaceutical compositions comprising one or more of the fluoro analogs can be administered to a patient for this purpose. Such compositions, which may contain acceptable diluents and/or carriers, can be prepared by reference to general texts in the field such as, for example, *Remington's Pharmaceutical Sciences*, Ed. Arthur Osol, 16th ed., 1980, Mack Publishing Co.

Contemplated equivalents of the general formulas set forth above for the DNJ analogs and derivatives as well as the intermediates are compounds otherwise corresponding thereto and having the same general properties wherein one or more of the various R groups are simple variations of the substituents as defined therein, e.g., wherein R is a higher alkyl group. In addition, where a substituent is designated as, or can be, a hydrogen, the exact chemical nature of a substituent which is other than hydrogen at that position is not critical so long as it does not adversely affect the overall activity and/or synthesis procedure.

The chemical reactions described above are generally disclosed in terms of their broadest application to the preparation of the compounds of this invention. Occasionally, the reactions may not be applicable as described to each compound included within the disclosed scope. The compounds for which this occurs will be readily recognized by those skilled in the art. In all such cases, either the reactions can be successfully performed by conventional modifications known to those skilled in the art, e.g., by appropriate protection of interfering groups, by changing to alternative conventional reagents, by routine modification of reaction conditions, and the like, or other reactions disclosed herein or otherwise conventional, will be applicable to the preparation of the corresponding compounds of this invention. In all preparative methods, all starting materials are known or readily preparable from known starting materials.

Without further elaboration, it is believed that one skilled in the art can, using the preceding description, utilize the present invention to its fullest extent. The following preferred specific embodiments are, therefore, to be construed as merely illustrative,

All reagents were used as received without purification. Methanol, toluene, benzaldehyde and triethylamine were dried over 3Å molecular sieves. Methylene chloride and tetrahydrofuran were purchased as anhydrous grade from Aldrich Chemical Co. and used as received. All proton and carbon NMR spectra were obtained on either a Varian VXR-300 or VXR-400 nuclear magnetic resonance spectrometer.

### Example 1

### Preparation of N-Carbobenzoxy-4,6-O-benzylidene-1- deoxynojirimycin.

A total of 75.0 g (0.46 moles) of 1-deoxynojirimycin was dissolved in 1500 mL of saturated aqueous sodium bicarbonate and then treated with 73.5 mL (87.8 g, 0.52 moles) of 95% benzyl chloroformate at room temperature using an overhead stirrer under a nitrogen atmosphere for eighteen hours. The solution was extracted once with 250 mL of methylene chloride to remove any benzyl chloride and unreacted benzyl chloroformate. The aqueous solution was then extracted ten times with 500 mL of ethyl acetate. After drying over anhydrous magnesium sulfate, filtering and removal of solvent, 102.8 g (76% yield) of a colorless oil was obtained which was identified as N-carbobenzoxy-1-deoxynojirimycin of sufficient purity for use in the next step; 300 MHz ¹H NMR (δ, CD₃OD) 7.40-7.20 (m, 5H), 5.15 (s, 2H), 4.23 (br m, 1H), 4.05 (br d, J=8.0 Hz, 1H) 3.87 (dd, J=4.0 and 6.0 Hz, 1H), 3.85-3.d78 (m, 2H), 3.78-3.70 (m, 2H), and 3.45 (br d, J=8.0 Hz, 1H).

To 102 g (0.345 mol) of N-carbobenzoxy-1-deoxynojirimycin, which had been dried in vacuo over phosphorous pentoxide overnight, was added 1000 mL of benzaldehyde (dried with 3 Å molecular sieves). This was warmed at 40°C while swirling on a rotary evaporator (no vacuum) until the oil was fully dissolved, then split in half and each half transferred to a 5 L three-necked flask and an additional 200 mL of benzaldehyde used to rinse the flask and 100 mL added to each reaction. After placing each reaction flask under nitrogen, 101 g of freshly activated 3 Å molecular sieves were added and then 257.6 g of anhydrous zinc chloride (dried in vacuo overnight over P₂O₅) was added and some warming observed. After stirring for five hours at room temperature, 1000 mL of ethyl acetate was added, each flask cooled in an ice bath and then 1500 mL of a cold saturated aqueous solution of sodium bicarbonate was added. Some foaming was observed. The white precipitate which formed was filtered and washed with ethyl acetate. The filtrate was separated and the organic layer washed with saturated sodium chloride, dried with magnesium sulfate and filtered. The organic layer from each reaction were combined and stripped at 40°C to afford a benzaldehyde solution of the desired product. This was then poured into 10 L of hexane with stirring, the precipitate collected and washed with hexane and air dried. This material was dissolved in approximately 1200 mL of hot ethyl acetate, hexane added to the cloud point (approx. 1500 mL), where-upon crystallization occurred. After cooling to room temperature, the precipitate was collected and washed well with hexane to afford 91.1 g (68%) of N-carbobenzoxy-4,6-O-benzylidene-1-deoxynojirimycin as a white solid, mp 147-148°C; 300 MHz ¹H NMR (δ, CD₃OD) 7.53-7.28 (m, 10H), 5.61 (s, 1H), 5.14 (s, 2H), 4.77 (dd, J_{5,6}=4.6 Hz, J_{6,6′}=11.0 Hz, 1H, H₆), 4.38 (t, J_{5,6′}=J_{6,6′}=11.0 Hz, 1H, H_{6′}), 4.16 (dd, J_{1,2}=4.2 Hz, J_{1,1′}=13.4 Hz, 1H, H₁), 3.69-3.50 (complex m, 3H, H₂, H₃ and H₄), 3.35 (ddd, J_{4,5}=J_{5,6′}=11.0 Hz, J_{5,6}=4.6 Hz, 1H, H₅) and 2.97 (dd, J_{1′,2}9.3 Hz, J_{1,1′}=13.4 Hz, 1H, H_{1′}); 75 MHz ¹³C NMR (CD₃OD) 156.7, 139.4, 138.0, 129.9, 129.7, 129.3, 129.2, 129.1, 127.6, 102.8, 81.9, 77.5, 71.5, 70.6, 68.6, 55.9 and 50.5 ppm; mass spectrum (m/e) 386 (M + H), 361, 327 and 280; and Anal. Calcd. for C₂₁H₂₃NO₆: C (65.45), H (6.01) and N (3.63); Found C (65.41), H (6.19) and N (3.59).

### Example 2

### Preparation of N-Butyryl-4-6-O-benzylidene-1-deoxynojirimycin

To a solution of 44.5 g (0.79 moles) of potassium hydroxide in 425 mL of methanol and 155 mL of water, was added 45 g (0.12 moles) of N-carbobenzoxy-4,6-O-benzylidene-1-deoxynojirimycin and the mixture refluxed under a nitrogen atmosphere for sixty-seven hours. After cooling to room temperature, the methanol was removed under reduced pressure, the residue transferred to a 2 L 3-necked flask and 100 mL of tetrahydrofuran added. The suspension was vigorously stirred, cooled in ice and 15.5 mL (15.9 g, 0.15 mol, 1.24 equiv.) of butyryl chloride added over ten minutes. After removal of the ice bath, the reaction was stirred at room temperature and monitored by tlc on silica gel using (20% v:v) methanol/methylene chloride as eluent. After 3 hours, an additional 3 mL of butyryl chloride was added and this was repeated 3xs until tlc indicated complete disappearance of the amine. The reaction was cooled in ice and acidified to neutral pH with 1 N hydrochloric acid. The tetrahydrofuran was removed under reduced pressure and the aqueous layer extracted twice with 700 mL of methylene chloride. After combining, the organic layers were washed with saturated aqueous sodium bicarbonate, dried over anhydrous magnesium sulfate, filtered and concentrated to afford 49.3 g of a clear oil. This was chromatographed on a Waters Prep 500A chromatogram using two silica gel cartridges and eluting first with 50:50 (v:v) ethyl acetate/-hexane and then ethyl acetate. In this manner one obtains 33.1 g (88% yield) of a clear colorless oil which was identified as N-butyryl-4,6-O-benzylidene-1-deoxynojirimycin. A sample was prepared for elemental analysis by rechromatography on a chromatatron using silica gel and eluting with ethyl acetate, stripping and drying under vacuum over phosphorous pentoxide; 300 MHz ¹H NMR (δ CDCl₃) 7.55-7.33 (m, 5H), 5.54 (s, 1H), 4.87 (dd, J_{5,6}=4.5 Hz, J_{6,6′}=11.3 Hz, 1H, H₆), 4.61 (t, J_{5,6′}=J_{6,6′}=11.3 Hz, 1H, H₆′), 3.95 (br s, 1H, OH), 3.79 (d, J=3.3 Hz, 1H, OH), 3.72 (dd, J_{1,2}=4.2 Hz, J_{1,1′}=13.8 Hz, 1H, H₁), 3.70-3.41 (complex m, 3H, H₂, H₃ and H₄), 3.21 (br ddd, J_{5,6}=4.5 Hz, J_{4,5}=J_{5,6′}=11.3 Hz, 1H, H₅), 2.78 (dd, J_{1′,2}=9.3 Hz, J_{1,1′}=13.8 Hz, 1H, H₁′), 2.32-2.13 (m, 2H), 1.61 (sextuplet, J=7.4 Hz, 2H) and 0.97 (t, J=7.4 Hz, 3H); 75 MHz ¹³C NMR (CDCl₃) 173.5, 137.4, 129.3, 128.3, 126.4, 101.6, 79.5, 77.1, 70.1, 69.5, 56.4, 49.9, 36.7, 18.4 and 13.9 ppm; mass spectrum (m/e) 328 (M+Li); and Anal. Calcd. for C₁₇H₂₃NO₅: C(63.53), H(7.23) and N(4.36); Found C(63.29), H(7.33) and N(4.31).

### Example 3

### Preparation of N-Carbobenzoxy-4-6-O-benzylidene-2,3-O-(di-n- butylstannylene)-1-deoxynojirimycin.

To a mixture of 0.50 g (1.30 mmol) of N-carbobenzoxy-4,6-O-benzylidene-1-deoxynojirimycin and 0.34 g (1.36 mmol) of di-n-butyltin oxide (both dried in vacuo over P₂O₅ overnight), under a nitrogen atmosphere, was added 5 mL of dry methanol (dried over 3Å molecular sieves) and the mixture refluxed for two hours. After cooling to room temperature, the volatiles were removed under vacuum, toluene added and then removed twice to afford N-carbobenzoxy-2,3-O-(di-n-butylstannylene)-4,6-O-benzylidene-1-deoxynojirimycin as a white solid; 300 MHz ′H NMR (δ, CDCl₃) 7.50-7.25 (m, 10H), 5.42 (s, 1H), 5.05 (AB quartet, J_{AB}=12.3 Hz, u_{AB}=14.2 Hz, 2H), 4.80 (dd, J_{6,6′}=11.8 Hz, J_{5,6}=4.5 Hz, 1H, H₆), 4.56 (dd, J_{6,6′}=11.8 Hz, J_{5,6′}=11.4 Hz, 1H, H_{6′}), 4.39 (dd, J_{1,1′}=12.7 Hz, J_{1,2}=4.1 Hz, H₁), 3.51 (dd, J_{3,4}=9.0 Hz, J_{4,5}=9.0 Hz, 1H, H₄), 3.29 (ddd, J_{5,6}=4.4 Hz, J_{4,5}=J_{5,6′}=10.5 Hz, 1H, H₅), 3.17-3.03 (m, 2H, H₂ and H₃), 2.62 (dd, J_{1.1′}=12.7 Hz, J_{1′,2}=10.2 Hz, 1H, H₁′) and 1.60-0.76 (m, 18H); and mass spectrum (m/e) 624 (M + Li).

### Example 4

### Preparation of N-Carbobenzoxy-2-O-(p-toluenesulfonyl)-4-6-O-benzylidene-1-deoxynojirimycin.

A mixture of 25.8 g (66.9 mmol) of N-carbobenzoxy-4, 6-O-benzylidene-1-deoxynojirimycin and 17.5 g (70.2 mmol, 1.05 equiv.) of di-n-butyltin oxide, both previously dried in vacuo over phosphorous pentoxide, and 260 mL of anhydrous methanol were refluxed under a nitrogen atmosphere for two hours. The methanol was removed, toluene was added and removed in vacuo. The residue was dissolved in 250 mL of anhydrous methylene chloride under nitrogen, 7.71 g (76.3 mmol, 1.14 equiv.) of triethylamine added and then a solution of 13.39 g (70.2 mmol, 1.05 equiv.) of recrystallized p-toluenesulfonyl chloride in 50 mL of anhydrous methylene chloride was added dropwise over ten minutes. After stirring for twenty hours, 260 mL of saturated aqueous sodium bicarbonate solution was added and the tin salts filtered (with difficulty). The organic layer was separated, washed with saturated sodium chloride, dried with anhydrous magnesium sulfate, filtered and concentrated to afford 38.7 g of a foam. This was chromatographed on a Waters Prep 500A Chromatogram using two silica gel cartridges and 50:50 (v:v) ethyl acetate/hexane as eluant to afford 34.0 g (94%) of a white foam, which was identified as N-carbobenzoxy-2-O-(p-toluenesulfonyl)-1-deoxynojirimycin. An analytical sample was prepared by recrystallization from ethyl acetate/hexane, mp 115-117°C; 300 MHz ¹H NMR (δ, CDCl₃) 7.82 (d, J=7.8 Hz, 2H), 7.50-7.35 (m, 10H), 7.31 (d, J=7.8 Hz, 2H), 5.51 (s, 1H), 5.12 (s, 2H), 4.76 (dd, J_{5,6}=4.5 Hz, J_{6,6′}=11.4, 1H, H₆),4.38 (ddd, J_{1′,2}=9.3 Hz, J_{1,2}=4.8 Hz, J_{2,3}=7.6 Hz, 1H, H₂), 4.32 (t, J_{6,6′}=11.4 Hz, J_{5,6′}= 9.5 Hz, 1H, H₆′) , 4.31 (dd, J_{1,2}=4.8 Hz, J_{1,1′}=13. 6 Hz, 1H, H₁), 3.78 (dt, J_{2,3}=J_{3,4}=9.4 Hz, J_{3,OH} =2.6 Hz, 1H, H₃), 3.59 (t, J_{3,4}=J_{4,5}=9.4 Hz, 1H, H₄) , 3.26 (ddd, J_{4,5}=9.4 Hz, J_{5,6}=4.5 Hz, J_{5,6′}=11.4 Hz, 1H, H₅), 3.04 (dd, J_{1′,2}=9.3 Hz, J_{1,1′}=13.6 Hz, 1H, H_{1′}), 2.63 (d, J_{3,OH}=2.6 Hz, 1H, OH) and 2.41 (s, 3H); 75 MHz ¹³C NMR (CDCl₃) 154.8, 145.2, 137.0, 135.8, 133.2, 129.8, 129.3, 128.7, 128.4, 128.3, 128.1, 126.2, 101.8, 79.9, 78.1, 73.9, 69.2, 67.8, 54.2, 47.1 and 21.7 ppm; mass spectrum (m/e) 546 (M + Li) and 374; and Anal. Calcd. for C₂₈H₂₉NO₈S: C (62.32), H (5.42) and N (2.66); Found C (62.65), H (5.40) and N (2.62).

### Example 5

### Preparation of N-Carbobenzoxy-2,3-anhydro-1-deoxy mannojirimycin, Method A

To 7.40 g of an 80% sodium hydride in oil dispersion (5.90 g, 247 mmol) under a nitrogen atmosphere, was added 280 mL of anhydrous tetrahydrofuran. After brief stirring, the solids were allowed to settle and the solution withdrawn via syringe. To this was then added a solution of 32.4 g (60 mmol) of N-carbobenzoxy-2-O-(p-toluenesulfonyl)-4,6-O-benzylidene-1-deoxynojirimycin in 350 mL of anhydrous tetrahydrofuran. After stirring at room temperature for eight and one-half hours, the slurry was slowly poured into a solution of 25 mL of acetic acid in 1400 mL of water under a nitrogen atmosphere. The resulting mixture was extracted with ethyl acetate, separated and the organic layer washed with saturated aqueous sodium bicarbonate, saturated aqueous sodium chloride, dried with magnesium sulfate, filtered and concentrated to afford 24.0 g of a slightly yellow-colored solid. This was dissolved in methylene chloride and hexane added to induce crystallization. The resulting white crystals were collected, washed with hexane and air-dried to afford 20.0 g (91% yield) of N-carbobenzoxy-2,3-anhydro-4,6-O-benzylidene-1-deoxymannojirimycin, mp 104-105°C; 300 MHz ¹H NMR (δ, CDCl₃) 7.67-7.53 (complex m, 10H), 5.67 (s, 1H), 5.16 (s, 2H), 4.76 (br s, 1H, H_{6′}), 4.59 (d, J_{1,1′}=15.0 Hz, 1H, H₁), 4.02 (dd, J_{5,6}=4.0 Hz, J_{6,6′}=11.4 Hz, 1H, H₆), 4.08 (*, J_{4,5}=10.0 Hz, 1H, H₄), 3.46 (dd, J_{1′,2}=0.9 Hz, J_{1,1′}=15.0 Hz, 1H, H_{1′}), 3.40 (d, J_{2,3}=3.0 Hz, 1H, H₂ or H₃), 3.25 (d, J_{2,3}=3.0 Hz, 1H, H₂ or H₃) and 3.10 (ddd, J_{5,6}=4.0 Hz, J_{5,6′}=J_{3,4}=10.0 Hz, 1H, H₅); 75 MHz ¹³C (CDCl₃) 156.2, 137.8, 136.6, 129.7, 129.1, 128.9, 128.8, 128.5, 126.6, 102.8, 73.0, 70.4, 68.0, 56.0, 54.7, 50.4 and 46.6 ppm; mass spectrum (m/e) 374 (M + Li); and Anal. Calcd. for C₂₁H₂₁NO₅ : C (68.64), H (5.77) and N (3.81); Found C (68.42), H (5.89) and N (3.77).

### Example 6

### Preparation of N-carbobenzoxy-2,3-anhydro-4,6-O-benzylidene-1-deoxymannojirimycin, Method B

To a mixture of 0.385 g (0.10 mmol) of N carbobenzoxy-4,6-O-benzylidene-1-deoxynojirimycin and 0.267 g (0.105 mmol) of dibutyltin oxide, under nitrogen atmosphere, was added 5 mL of dry methanol and the mixture refluxed for two hours. After cooling, the methanol was removed in vacuo and further dried with two toluene azeotropes. The crude stannylene was dissolved in 10 mL of dry methylene chloride and placed under nitrogen atmosphere and cooled to -78°C. To this was added 100 µL dry pyridine and 178 µL of trifluoromethanesulfonic anhydride, and the reaction allowed to warm to room temperature over 16 hours. The mixture was diluted with 25 mL of methylene chloride and extracted 2 x 20 mL with saturated sodium bicarbonate. The organic phase was dried over MgSO₄, filtered, and conc. in vacuo to yield after silica gel chromatography using 1% CH₃OH, 99% CH₂Cl₂, 130 mg (36% yield) of a white solid which was identified as N-carbobenzoxy-2,3-anhydro-4,6-O-benzylidene-1-deoxynojirimycin, mp 104-105°C.

### Example 7

### Preparation of N-Carbobenzoxy-4,6-O-benzylidene-1,2-dideoxy-2-fluoronojirimycin

In a 250 mL round-bottom flask was placed 14.67 g (39.9 mmol) of N-carbobenzoxy-2,3-anhydro-1-deoxymannojirimycin and 29.34 g(196 mmol, 4.9 equiv.) of diisopropylamine trihydrofluoride. The flask was then placed on a rotary evaporator under a nitrogen atmosphere and with swirling immersed in an oil bath maintained at 125°C. After swirling for seventy hours, the flask was cooled and the mixture dissolved in ethyl acetate and saturated aqueous sodium bicarbonate solution. After separating, the organic layer was washed with 0.2 N hydrochloric acid, saturated aqueous sodium chloride, dried over anhydrous magnesium sulfate, filtered and stripped to afford 14.2 g of a brown oil, whose ¹H and ¹³C NMR spectra were consistent with the presence of two isomeric fluorohydrins in a 2.8:1 ratio. The crude material was chromatographed on a Waters Prep 500A chromatogram using two silica gel cartridges and first 100% methylene chloride and then 2% (v:v) methanol/methylene chloride as eluant. The first isomer to elute (5.67 g, 37%) corresponded to the major isomer. It was recrystallized from chloroform/hexane to afford 4.30 g (28%) of a white solid which was identified as N-carbobenzoxy-4, 6-O-benzylidene-1,2-dideoxy-2-fluoronojirimycin, mp 94-95°C; 300 MHz ¹H NMR (δ, CDCl₃) 7.57-7.34 (m, 10H), 5.57 (s, 1H), 5.17 (AB quartet, J_{A,B}=12.1 Hz, u_{A,B}=15.0 Hz, 2H, CBZ), 4.87 (dd, J_{5,6}=4.6 Hz, J_{6,6′}=11.3 Hz, 1H, H₆), 4.53 (dddd, J=_{1,2}4.6 Hz, J_{1′,2}=8.7 Hz, J_{2,3}=6.6 Hz, J_{2,F}=48.4 Hz, 1H, H₂), 4.26 (t, J_{5,6′}=J_{6,6′}=11.3 Hz, 1H, H_{6′}), 4.25 (ddd, J_{1,2}=4.6 Hz, J_{1,1′}=14.1 Hz, J_{1,F}=13.3 Hz, 1H, H₁), 3.88 (ddd J_{2,3}=6.6 Hz, J_{3,4}=9.6 Hz, J_{3,F}=18.3 Hz, 1H, H₃), 3.65 (t, J_{3,4}=J_{4,5}=9.6 Hz, 1H, H₄), 3.36 (ddd, J_{5,6}=4.6 Hz, J_{5,6′}=10.2 Hz, J_{4,5}=9.6 Hz, 1H, H₅), 3.26 (ddd, J_{1,2}=8.7 Hz, J_{1,1′}=14.1 Hz, J_{1′,F}=6.0 Hz, 1H, H_{1′}) and 3.06 (br s, 1H, OH); 75 MHz ¹³C NMR (CDCl₃) 154.9, 137.0, 135.7, 129.3, 128.6, 128.4, 128.3, 128.1, 126.2, 101.7, 89.5 (d, J_{C2,F}=180.5 Hz, C₂), 79.6 (d, J_{C4,F}=8.6 Hz, C₄), 74.4 (d, J_{C3,F}=22.0 Hz, C₃), 69.3, 67.8, 53.4 and 46.1 (d, J_{C1,F}=27.4 Hz, C₁) ppm; mass spectrum (m/e) 388 (M + H), 282 and 238; and Anal. Calcd. for C₂₁H₂₂FNO₅: C (65.10), H (5.74) and N (3.61); Found C (65.37), H (5.82) and N (3.69).

### Example 8

### Preparation of 1,2-Dideoxy-2-fluoronojirimycin.

In a Fisher-Porter bottle was placed 3.90 g (10.1 mmol) of N-carbobenzoxy-4,6-O-benzylidene-1,2-dideoxy-2-fluoronojirimycin, 38mL of methanol and 10 mL of water. To the homogeneous solution under a nitrogen atmosphere was added 3.9 g of a 10% palladium on carbon catalyst. The reactor was sealed, flushed 3 times with 2.8 Kg/sq. cm. of nitrogen and 4 times with 3.5 Kg/sq. cm. of hydrogen. The reactor was charged with 3.5 Kg/sq. cm. of hydrogen and heated at 50°C for twenty-one hours. After cooling, the reactor was flushed with nitrogen, opened and the contents filtered through celite, which was then washed with water. The filtrate was extracted once with ethyl acetate to remove organics and the aqueous layer was concentrated under vacuum at 50°C to afford an oil. The remaining water was removed by azeotrope with ethanol to provide 1.2 g of a white solid, whose ¹H NMR was consistent with 1,2-dideoxy-2-fluoronojirimycin. This was dissolved in water, filtered to remove a slight grey color and stripped again. This was recrystallized by dissolving in a minimal amount of water, followed by ethanol and then hexane to afford 0.58 g (35%) of a white solid, which was identified as 1,2-dideoxy-2-fluoronojirimycin, mp 161.5-162.5°C; 400 MHz ¹H NMR (δ, D₂O) 4.38 (dddd, J_{1′},₂=5.5 Hz, J_{1,2}=10.8 Hz, J_{2,3}=9.5 Hz, J_{2,F}=50.5 Hz, 1H, H₂) , 3.83 (dd, J_{5,6}=3.0 Hz, J_{6,6′}=11.7 Hz, 1H, H₆), 3.64 (dd, J_{5,6′}=6.0 Hz, J_{6,6′}=11.7 Hz, 1H, H_{6′}), 3.62 (dt, J_{2,3}=J_{3,4}=9.5 Hz, J_{3,F}=14.5 Hz, 1H, H₃), 3.39 (ddd, J_{1,2}=5.5 Hz, J_{1,1′}=12.3 Hz, J_{1,F}=1.5 Hz, 1H, H₁), 3.28 (t, J_{3,4}=J_{4,5}=9.5 Hz, 1H, H₄), 2.65 (ddd, J_{1,1′}=12.3 Hz, J_{1′,2}=10.7 Hz, J_{1′,F}=4.9 Hz, 1H, H₁′) and 2.56 (ddd, J_{5,6}=3.0 Hz, J_{5,6′}=6.0 Hz, J_{4,5} = 9.5 Hz, 1H, H₅); 75 MHz ¹³C NMR (D₂O) 94.6 (d, J_{C2,F}=176.7 Hz, C₂), 79.9 (d, J_{C3,F}=16.7 Hz, C₃), 74.3 (d, J_{C4,F}=8.6 Hz, C₄), 64.3, 63.5 and 49.3 (d, J_{C1′F}=24.0 Hz, C₁) ppm; mass spectrum (m/e) 166 (M+H), 148 and 134; and Anal. Calcd. for C₆H₁₂FNO₃: C (43.63), H (7.34) and N (8.48); Found C (43.79), H (7.40) and N (8.37).

### Example 9

### Preparation ofN-Butyryl-2-O-(p-toluenesulfonyl)-4,6-O-benzylidene-1-deoxynojirimycin

In a 1L round-bottom flask was placed 27.11 g (84 mmol) of N-butyryl-4,6-O-benzylidene-1-deoxynojirimycin (previously dried under vacuum over phosphorous pentoxide), 370 mL of dry toluene and then 22.05 g (88 mmol, 1.05 equiv.) of dibutyltin oxide. The mixture was refluxed under a nitrogen atmosphere with azeotrope removal of water for two hours. The solution was cooled to room temperature, 9.63 g (95 mmol, 1.13 equiv.) of dry triethylamine added and then a solution of 17.69 g (93 mmol, 1.1 equiv.) of recrystallized p-toluenesulfonyl chloride in 45 mL of toluene over ten minutes. After stirring at room temperature for twenty-two hours, the toluene layer was washed with 1 N hydrochloric acid, dried over anhydrous magnesium sulfate, filtered and stripped. The residue was chromatographed on a Waters Prep 500A chromatogram using two silica gel cartridges and eluting first with a 20% (v:v) ethyl acetate/- hexane and then 50% ethyl acetate/hexane to elute the desired product (28.0 g, 70% yield) and then 100% ethyl acetate to recover starting material (4.4 g, 84% conversion). The desired product was identified as N-butyryl-2-O-(p-toluenesulfonyl)-4,6-O-benzylidene-1-deoxynojirimycin; 300 mHz ¹H NMR (δ, CDCl₃) 7.87 (d, J=8.3 Hz, 2H), 7.52-7.35 (m,7H), 5.54 (s, 1H), 4.88 (dd, J_{5,6}=4.5 Hz, J_{5,6′}=11.5 Hz, 1H, H₆), 4.49 (t, J_{5,6′}=J_{6,6′}=11.5 Hz, 1H, H₆′), 4.35 (ddd, J_{1,2}=4.4 Hz, J_{1′,2}=9.0 Hz, J_{2,3}=9.5 Hz, 1H, H₂), 4.04 (dd, J_{1,2}=4.4 Hz, J_{1,1′}=14.3 Hz, 1H, H₁), 3.78 (dt, J_{3,OH}=2.6 Hz, J_{2,3}=J_{3,4}=9.5 Hz, 1H, H₃), 3.64 (t, J_{3,4}=J_{4,5}=9.5 Hz, 1H, H₄), 3.33 (ddd, J_{4.5}=9.5 Hz, J_{5,6}=4.5 Hz, J_{5,6′}=11.5 Hz, 1H, H₅), 3.22 (dd, J_{1′,2}=9.0 Hz, J_{1,1′}=14.3 Hz, 1H, H₁′), 2.85 (br d, J_{3,OH}=2.6 Hz, 1H, OH), 2.36-2.18 (m, 2H), 1.64 (sextuplet, J = 7.5 Hz, 2H) and 0.99 (t, J=7.5 Hz, 3H); 75 MHz ¹³C NMR (CDCl₃) 173.4, 145.3, 137.1, 132.9, 129.8, 129.2, 128.2, 128.0, 126.1, 101.6, 79.3, 78.5, 73.6, 69.1, 55.4, 47.9, 36.3, 21.6, 18.3 and 13.7 ppm; and mass spectrum (m/e) 482 (M+Li), 476 (M+H) and 310.

### Example 10

### Preparation of N-Butyryl-2,3-anhydro-4,6-O-benzylidene- 1-deoxymannojirimycin

In a 1L three-necked flask equipped with an overhead stirrer was placed 7.22 of an 80% sodium in oil dispersion(5.8 g, 0.24 mol, 4.1 equiv.). After placing the flask under a nitrogen atmosphere, the sodium hydride was washed with 250 mL of anhydrous tetrahydrofuran, the solvent removed via a canula and the flask cooled in an ice bath. To this was added a solution of 28.0 g (59 mmol) of N-butyryl-2-O-(p-toluenesulfonyl)-4,6-O-benzylidene-1-deoxynojirimycin in 200 mL of anhydrous tetrahydrofuran over a ten minute period and the temperature maintained below 10°C. An additional 2 x 25 mL of anhydrous tetrahydrofuran was used to rinse the flask containing tosylate and added to the reaction. The ice bath was removed and the reaction stirred at room temperature for twenty-one hours. In a 3üL flask was placed 990 mL of water and 20 mL of acetic acid. This was cooled in ice and placed under a nitrogen atmosphere. To this was then slowly added the reaction mixture while maintaining the temperature below 15°C. A white precipitate was observed. This was dissolved in 500 mL of methylene chloride, the layers separated and the organic layer washed with saturated aqueous sodium bicarbonate, dried over anhydrous magnesium sulfate, filtered and stripped to afford 17.7 g of an off-white solid. This was recrystallized from methylene chloride and hexane to afford 15.6 g (87% yield) of pure product which was identified as N-butyryl-2,3-anhydro-4,6-O-benzylidene-1-deoxynojirimycin, mp 120-120.5°C; 300 MHz ¹H NMR(δ, CDCL₃) 7.58-7.49 (m, 2H), 7.46-7.37 (m, 3H), 5.71 (s, 1H), 5.34 (t, J_{5,6}=J_{6,6′}=11.0 Hz, 1H, H₆′), 4.50 (dd, J_{5,6}=4.0 Hz, J_{6,6′}=11.0 Hz, 1H, H₆) , 4.19 (br d, J_{1,1′}=14.9 Hz, 1H, H₁), 4.15 (d, J_{4,5}=10.1 Hz, 1H, H₄), 3.57 (d, J_{1,1}′=14.9 Hz, 1H, H₁'), 3.39 (d, J_{2,3}=3.5 Hz, 1H, H₃), 3.25 (br d, J_{2,3}=3.5 Hz, 1H, H₂), 3.05 (ddd, J_{4,5}=10.1 Hz, J_{5,6}=4.0 Hz, J_{5,6′}=11.0 Hz, 1H, H₅), 2.37-2.18 (m, 2H), 1.73-1.58 (m, 2H) and 0.99 (t, J=7.5 Hz, 3H); 75 MHz ¹³C NMR (CDCl₃) 174.6, 137.3, 129.1, 128.3, 126.1, 102.2, 71.2, 70.1, 57.6, 54.3, 49.7, 47.2, 36.6, 18.2 and 13.8 ppm; mass spectrum (m/e) 310 (M+Li); and Anal. Calcd. for C₁₇H₂₁NO₄: C (67.31), H (6.98) and N (4.62); Found C (67.15), H (7.28) and N (4.33).

### Example 11

### Preparation of N-Butyryl-4,6-O-benzylidene-1,2-dideoxy-2-fluoronojirimycin

In a 250 mL round-bottom flask were placed 15.24 g (50.2 mmol) of N-butyryl-2,3-anhydro-4,6-O-benzylidene-1-deoxymannojirimycin and 30.5 g (189 mmol, 6 equiv.) of diiospropylamine trihydrofluoride. The flask was then placed on a rotovary evaporator under a nitrogen atmosphere and with swirling immersed in an oil bath maintained at 125°C. After swirling for fifty-four hours, the flask was cooled and the mixture dissolved in ethyl acetate and saturated aqueous sodium bicarbonate solution. After separating the layers, the organic layer was washed with 0.2 N hydrochloric acid and saturated sodium chloride, dried over anhydrous magnesium sulfate, filtered and stripped to afford 12.2 g of crude material whose ¹H and ¹³C NMR indicated a 2.8:1 mixture of isomeric fluorohydrins. These were separated on a Waters Prep 500A chromatogram using two silica gel cartridges and first methylene chloride, followed by 2% (v:v) methanol/methylene chloride as eluant. The first isomer to elute (5.7 g, 35%), which corresponded to the major isomer, was identified as N-butyryl-4,6-O-benzylidene-1,2-dideoxy-2-fluoronojirimycin. It was recrystallized from chloroform and hexane to afford 6.0 g of material which contained chloroform in the crystals. This was suitable for the subsequent chemistry. An analytical sample was prepared by recrystallizing 200 mg from ethyl acetate/hexane to afford 143 mg of pure compound, mp 111°-112°C; 300 MHz ¹H NMR(δ, CDCl₃) 7.57-7.48 (m, 2H), 7.47-7.38 (m, 3H), 5.61 (s, 1H), 4.96 (dd, J_{5,6}=4.4 Hz, J_{6,6′}=11.3 Hz, 1H, H₆), 4.60 (dddd, J_{1,2}=4.1 Hz, J_{2,3}=5.7 Hz, J_{1′,2}=7.5 Hz, J_{2,F}=48.3 Hz, 1H, H₂) , 4.31 (t, J_{5,6′},=J_{6,6′}=11.3 Hz, 1H, H₆′) , 4.03-3.87 (m, 1H, H₃), 3.91 (ddd, J_{1,2}=4.1 Hz, J_{1,1′}=14.5 Hz, J_{1,F}= 16.3 Hz, 1H, H₁), 3.75 (t, J_{4,5}=J_{3,4}=9.7 Hz, 1H, H₄), 3.54 (ddd, J_{4,5}=9.7 Hz, J_{5,6}=4.4 Hz, J_{5,6}′=11.3 Hz, 1H, H₅), 3.45 (dd, J_{1′,2}=7.5 Hz, J_{1,1′}=14.6 Hz, 1H, H₁′), 2.43-2.25 (m, 2H), 1.67 (sextuplet, J=7.4 Hz, 2H) and 1.01 (t, J=7.4 Hz, 3H); 75 MHz ¹³C NMR (CDCl₃) 173.8, 137.2, 129.5, 128.5, 126.4, 102.1, 90.6 (d, J_{C2,F}=181.5 Hz, C₂), 79.3 (d, J_{C4,F}=9.0 Hz, C₄), 74.5 (d, J_{C3,F}=23.3 Hz, C₃), 69.4, 54.3, 46.7 (d, J_{C1,F}=29.3 Hz, C₁), 36.5, 18.5 and 13.9 ppm; mass spectrum (m/e) 330 (M+Li); and Anal. Calcd. for C₁₇H₂₂FNO₄; C (63.13), H (6.87) and N (4.33); Found C (62.98), H (6.80) and N (4.17).

### Example 12

### Preparation of N-Butyl-1,2-dideoxy-2-fluoronojirimycin

To a solution of 1.50 g (4.64 mmol) of N-butyryl-4, 6-O-benzylidene 1,2-dideoxy-2-fluoronojirimycin in 8.9 mL of anhydrous tetrahydrofuran under a nitrogen atmosphere of 0°C, was added 3 mL (30 mmol, 6.5 equiv.) of 10 M borane:methyl sulfide complex over a ten-minute period. The ice bath was removed and the reaction stirred at room temperature for four hours. After cooling to 0°C, 8.4 mL of anhydrous methanol was slowly added over ten minutes and stirring continued for an additional thirty minutes. The volatiles were removed under reduced pressure, the residue dissolved in 50 mL of methylene chloride, extracted twice with a saturated aqueous sodium bicarbonate solution, dried over anhydrous magnesium sulfate, filtered and stripped to afford 1.43 g of a clear oil. This was dissolved in a mixture of 15 mL of water and 15 mL of trifluoroacetic acid and stirred at room temperature for three hours. The volatiles were removed under reduced pressure, toluene added and removed, then methanol added and removed. The residue was triturated with diethyl ether. The residue was dried under vacuum to afford 1.0 g of an off-white solid. An Amberlite CG-400 (Cl⁻ form) resin was conditioned as follows; in an addition funnel, 25 mL of the resin was washed with 250 mL of aqueous 1 N sodium hydroxide solution and then with water until the pH stabilized at approximately 5. The crude product was dissolved in a minimal amount of water, applied to the column and eluted with 150 mL of water. Lyophilization of the eluant afforded 610 mg of material. This was then chromatographed on 23 g of Aldrich silica gel (230-400 mesh, 60Å) using 10% (v:v) ethanol/methylene chloride to afford 250 mg (24%) of pure material, which was recrystallized from ethanol/hexane to afford 210 mg (20% yield) of a white powder, mp 92°C; 300 MHz ¹H NMR(δ, D₂O) 4.44 (dddd, J_{1,2}=5.3 Hz, J_{1′,2}=11.0 Hz, J_{2,3}=9.1 Hz, J_{2,F}=50.5 Hz, 1H, H₂), 3.91 (dd, J_{5,6}=2.6 Hz, J_{6,6′}=12.8 Hz, 1H, H₆), 3.84 (dd, J_{5,6′}=2.6 Hz, J_{6,6′}=12.8 Hz, 1H, H₆′), 3.57 (dt, J_{2,3}=J_{3,4}=9.1 Hz, J_{3,F}=15.3 Hz, 1H, H₃), 3.43 (t, J_{3,4}=J_{4,5}=9.1 Hz, 1H, H₄), 3.24 (ddd, J_{1,2}=5.3 Hz, J_{1,1′}=11.0 Hz, J_{1,F}=4.1 Hz, 1H, H₁), 2 .83-2.61 (m, 2H, N-CH₂), 2.50 (ddd, J_{1′,2}=11.0 Hz, J_{1,1′}=11.0 Hz, J_{1′,F}=5.1 Hz, 1H, H_{1′}), 2.29 (dt, J_{5,6}=J_{5,6′}=2.6 Hz, J_{4,5}=9.1 Hz, 1H, H₅) 1.55-1.42 (m,2H), 1.31(sextuplet, J=7.0Hz, 2H) and 0.93 (t, J=7.0Hz,3H); 101 MHz ¹³C NMR (D₂O) 92.7 (d, J_{C2,F}=173.7 Hz, C₂), 79.5 (d, J_{C3,F}=17.0 Hz, C₃), 72.3 (d, J_{C4,F}=11.6 Hz, C₄), 67.6, 60.1, 55.3 (d, J_{C1,F}=25.4, C₁) 54.6, 28.0, 23.0 and 16.1 ppm) mass spectrum (m/e) 222 (M+H); and Anal. Calcd. for C₁₀H₂₀FNO₃: C (54.27), H (9.13) and N (6.32); Found C (54.54), H (9.42) and N (6.24).

### Example 13

### Preparation of N-Carbobenzoxy-4,6-O-benzylidene-2,3-O-(di-n-butylstannylene)-1-deoxynojirimycin.

To a mixture of 0.50 g (1.30 mmol) of N-carbobenzoxy-4,6-O-benzylidene-1 deoxynojirimycin and 0.34 g (1.36 mmol) of di-n-butyltin oxide (both dried in vacuo over P₂O₅ overnight), under a nitrogen atmosphere, was added 5 mL of dry methanol (dried over 3Å molecular sieves) and the mixture refluxed for two hours. After cooling to room temperature, the volatiles were removed under vacuum, toluene added and then removed twice to afford N-carbobenzoxy-2,3-O-(di-n-butylstannylene)-4,6-O-benzylidene-1-deoxynojirimycin as a white solid; 300 MHz ′H NMR (d, CDCl₃) 7.50-7.25 (m. 10H), 5.42 (s, 1H), 5.05 (AB quartet, J_{AB}=12.3 Hz, u_{AB}=14.2 Hz, 2H), 4.80 (dd, J_{6,6′}=11.8 Hz, J_{5,6}=4.5 Hz, 1H, H₆), 4.56 (dd, J_{6,6′}=11.8 Hz, J_{5,6′}=11.4 Hz, 1H, H_{6′}), 4.39 (dd, J_{1,1′}=12.7 Hz, J_{1,2}=4.1 Hz, H₁), 3.51 (dd, J_{3,4}=9.0 Hz, J_{4,5}=9.0 Hz,1H, H₄), 3.29 (ddd, J_{5,6}=4.4 Hz, J_{4,5}=J_{5,6′}=10.5 Hz, 1H, H₅), 3.17-3.03 (m, 2H, H₂ and H₃), 2.62 (dd, J_{1,1′}=12.7 Hz, J_{1′,2}=10.2 Hz, 1H, H_{1′}) and 1.60-0.76 (m, 18H); and mass spectrum (m/e) 624 (M + Li).

### Example 14

### Preparation of N-carbobenzoxy-2-O-benzoyl-4,6-O-benzylidene-1-deoxynojirimycin, Method A

A suspension of 30.0 g (77.8 mmol) of N-carbobenzoxy-4,6-O-benzylidene-1-deoxynojirimycin and 20.3 g (81.7 mmol) of di-n-butyltin oxide in 480 mL of dry toluene were heated with azeotropic removal of water for two hours, whereupon a homogeneous solution resulted. After cooling to room temperature, 9.43 g (93.4 mmol) of dry triethylamine and then 11.16 g (79.4 mmol) of benzoyl chloride were added. After stirring at room temperature for fifteen hours, an aqueous solution of saturated sodium bicarbonate was added, the solids filtered and washed with ethyl acetate. The filtrate was separated and the organic layer washed with 1N hydrochloric acid, dried with magnesium sulfate, filtered and concentrated to afford 38.1 g of a white solid. This was recrystallized from hot methylene chloride and hexane to afford 24.6 g (67%) of N-carbobenzoxy-2-O-benzoyl-4,6-O-benzylidene-1-deoxynojirimycin, mp 120-121°C; 300 MHz ¹H NMR (d, CDCl₃) 7.99 (d, J=7.7 Hz, 2H), 7.57 (t, J=7.7 Hz, 1H), 7.52-7.28 (complex m. 7H), 5.59 (s, 1H), 5.12 (s, 2H), 5.11-5.05 (m, 1H, H₂), 4.86 (dd, J_{5,6}=4.4 Hz, J_{6,6′}=11.1 Hz, 1H, H₆), 4.20 (t, J_{6,6′}=J_{5,6′}=11.1 Hz,1H, H_{6′}), 4.17 (dd, J_{1.2}=4.1 Hz, J_{1.1′}=14.0 Hz, 1H, H₁), 3.99 (dd, J_{2,3}=6.2 Hz, J_{3,4}=9.0 Hz, 1H, H₃), 3.84 (t, J_{3,4}=J_{4,5}=9.0 Hz, 1H, H₄), 3.50 (ddd, J_{4,5}=9.0 Hz, J_{5,6}=4.4 Hz, J_{5,6′}=11.1 Hz, 1H, H₅), 3.43 (dd, J_{1′,2}=7.7 Hz, J_{1,1′}=14.0 Hz, 1H, H_{1′}), and 2.81 (br s, 1H, OH), 75 MHz ¹³C NMR (CDCl₃) 165.9, 155.4, 137.2, 135.9, 133.5, 129.8, 129.4, 129.3, 128.6, 128.5, 128.4, 128.3, 128.1, 126.3, 102.0, 80.1, 74.1, 73.6, 69.5, 67.7, 53.1 and 45.2 ppm; mass spectrum (m/e) 496 (M + Li); and Anal. Calcd. or C₂₈H₂₇NO₇: C (68.70), H (5.55) and N (2.86); Found C (68.88), H (5.64) and N (2.70).

### Example 15

### Preparation of N-Carbobenzoxy-2-O-benzoyl-4-6-O-benzylidene-1-deoxynojirimycin, Method B

The stannylene intermediate from Example 2 (1.30 mmol) was placed under a nitrogen atmosphere, 5 mL of anhydrous methylene chloride was added, followed by 0.20 mL (150 mg, 1.48 mmol, 1.14 eq) of dry triethylamine and then 0.15 mL (183 mg, 1.30 mmol, 1.0 eq) of benzoyl chloride. After stirring at room temperature for one hour and fifteen minutes, an aqueous saturated sodium bicarbonate solution was added, the layer separated, the organic layer washed with IN hydrochloric acid, dried with magnesium sulfate, filtered and concentrated to afford 0.93 g of crude material. This was chromatographed on a 2 mm silica gel chromatatron plate using methylene chloride, 1% methanol/methylene chloride and 2% methanol/methylene chloride to afford 0.45 g (73%) of N-carbobenzoxy-2-O-benzoyl-4,6-O-benzylidene-1-deoxynojirimycin as a white solid, mp 118-120°C, whose spectra were identical to the material of Example 3.

### Example 16

### Preparation of N-Carbobenzoxv-2-O-benzoyl-4,6-O-benzylidene-1,5-dideoxy-1,5-imino-D-allitol.

A 250 ml three-necked round-bottomed flask equipped with a nitrogen inlet, overhead stirrer, and rubber septum, was charged with 2.45 g (30.9 mmol) of distilled methyl sulfoxide and 50 mL of dry dichloromethane and cooled to -60°C with a dry ice/acetone bath. To this was added dropwise over ca. 20 min. 5.63 g (26.8 mmol) of trifluoroacetic anhydride (a white precipitate should form). After an additional 10 min. of stirring was added a solution of 10.10 g (20.6 mmol) of N-carbobenzoxy-2-O-benzoyl-4,6 O-benzylidene-1-deoxynojirimycin in 50 mL of dichloromethane at a rate which maintains a -60°C reaction temperature. The reaction was stirred an additional 50 min. at which time the bath was removed and the reaction was quenched immediately with 11.8 mL of triethylamine. The solution was allowed to war to 0°C and poured into 50 mL of dichloromethane. The solution was washed with 1.0 M HCl, saturated sodium bicarbonate and saturated brine, dried over magnesium sulfate, filtered and concentrated in vacuo, azeotroped with toluene to yield 9.90 g of a white foamy oil which was identified as N-carbobenzoxy-2-O-benzoyl-4,6-O-benzylidene-3-keto-1-deoxynojirimycin; 300 MHz ¹H NMR (d, CDCl₃) 8.10-7.30 (m, 15H), 5.67 (s, 1H), 5.54 (dd, J_{1,2}=5.8 Hz, J_{1′,2}=9.3 Hz, 1H, H₂), 5.22 (dd, g_{AB}=16.1 Hz, J_{AB}=11.7 Hz, 2H, Z CH₂), 4.86 (dd, J_{5,6}=5.6 Hz, J_{6,6′}=11.3 Hz, 1H, H₆), 4.70 (dd, J_{1,2}=5.7 Hz, J_{1,1′}=13.7 Hz, 1H, H₁), 4.65 (d, J_{4,5}=9.4 Hz, 1H, H₄), 4.60 (t, J_{5,6′}=10 Hz, J_{6,6′}=11.3 Hz, 1H, H_{6′}), 3.74 (dt, J_{4,5}=9.4 Hz, J_{5,6′}=5.6 Hz, J_{5,6′}=10.0 Hz, 1H, H₅) and 3.62 (dd, J_{1,1′}=13.7 Hz, J_{1′,2}=9.3 Hz, 1H, H_{1′}).

The crude ketone was used without further purification. It was dissolved in 50 mL of methanol and 400 mL of tetrahydrofuran was added. The solution was cooled to -5°C in an ice bath and 1.2 mL of acetic acid was added followed by 0.770 g (20.6 mmol) of sodium borohydride and stirred for 10 min. at which time another 0.770 g of sodium borohydride was added and stirred an additional 1 min. The reaction was quenched with 100 mL of saturated ammonium chloride and diluted with 25 mL of water to dissolve precipitate. The solution was extracted with 3 x 200 mL of ethyl acetate. The organic layer was dried over magnesium sulfate, filtered and concentrated in vacuo to yield a mixture of inverted allitol to glucitol of 2:1 with less than 10% migrated 3-O-benzoylallitol. The crude was subjected to SiO₂ chromatography, using dichloromethane: ethylacetate (95:5) as eluant to yield 3.36 g of desired allitol which was recrystallized from dichloromethane: hexanes to yield 3.36 g (34% yield) of white crystals which were identified as the desired allitol derivative, mp 143-144°C; 300 MHz ¹H NMR (d, CDCl₃) 8.08-7.30 (m, 15H), 5.56 (s, 1H), 5.13 (dd, g_{AB}=21.2 Hz, J_{A,B}=12.5 Hz, 2H, Z CH₂), 5.08 (ddd, J_{1,2}=5.0 Hz, J_{1′,2}=11.8 Hz, J_{2,3}=3.0 Hz, 1H, H₂), 4.89 (dd, J_{5,6}=4.4 Hz, J_{6,6′}=11.7 Hz, 1H, H₆), 4.56 (t, J_{5,6′}=11.0 Hz, J_{6,6′}=11.7 Hz, 1H, H_{6′}), 4.50 (br s, 1H, H₃), 4.31 (dd, J_{1,2}=5.0 Hz, J_{1,1′}=12.7 Hz, 1H, H₁), 3.85 (dd, J_{3,4}=1.9 Hz, J_{4,5}=9.8 Hz, 1H, H₄), 3.78 (dt, J_{5,6}=4.4 Hz, J_{4,5}=9.8 Hz, J_{5,6′}=11.0 Hz, 1H, H₅) and 3.45 (dd, J_{1′,2}=11.8 Hz, J_{1,1′}=12.7 Hz, 1H, H_{1′}); 75 MHz ¹³C NMR (CDCl₃) 165.5, 154.9, 137.2, 136.1, 133.4, 129.9, 129.6, 129.3, 128.6, 128.4, 128.3, 128.2, 126.1, 101.4, 77.9, 69.6, 68.9, 67.7, 67.6, 50.6, 43.6 ppm 496 (M + Li).

### Example 17

### Preparation of N-Carbobenzoxy-2-O-benzoyl-4,6-O-benzylidene-1,3-dideoxy-3-fluoronojirimycin.

To a solution of 3.31 g (6.7 mmol) of N-carbobenzoxy-2-O-benzoyl-4,6-O-benzylidene-1,5-dideoxy-1,5-iino-D-allitol in 20 mL of dichloromethane, 1.11 ml (14 mmol) of pyridine was added, the solution was cooled to -78°C and 2.74 mL (20.7 mmol) of diethylaminosulfur trifluoride (DAST) was added dropwise over 5 min. The bath was removed and the reaction warmed gently to reflux for 16 hours, cooled to room temperature and quenched with saturated sodium bicarbonate. The solution was extracted with dichloromethane and washed with 1N HCl, saturated sodium bicarbonate, and brine, dried, filtered and concentrated. This was chromatographed on silica gel using 10% ethyl acetate/hexane as eluant, the desired fractions combined and recrystallized from methylene chloride/hexane to afford 1.92 g (60% yield) of N-carbobenzoxy-2-O-benzoyl-4,6-O-benzylidene-1,3-dideoxy-3-fluoronojirimycin as white needles, mp. 143-144°C; 400 MHz ¹H NMR (d, CDCl₃) 8.01 (d, J=8.0 Hz, 2H), 7.60-7.25 (m, 13H), 5.61 (s, 1H), 5.31 (dddd, J_{1,2}=3.7 Hz, J_{1′,2}=8.0 Hz, J_{2,3}=5.9 Hz, J_{2,1}=17.2 Hz, 1H, H₂), 5.10 (s, 2H), 4.89 (ddd, J_{5,6}=4.4 Hz, J_{6,6′}=10.7 Hz, J_{6,F}=1.7 Hz, 1H, H₆), 4.80 (dt, J_{3,F}=51.1 Hz, J_{2,3}=5.9 Hz, J_{3,4}=8.5 Hz, 1H, H₃), 4.27 (t, J_{5,6′}=10.3 Hz, J_{6,6′}=10.7 Hz, 1H, H_{6′}), 4,22 (ddd, J_{1,2}=3.7 Hz, J_{1,1′}=13.9 Hz, J_{1,F}=2.4 Hz, 1H, H₁), 4.09 (ddd, J_{3,4}=8.5 Hz, J_{4,5}=10.5 Hz, J_{4,F}-18.7 Hz, 1H, H₄), 3.51 (ddd, J_{5,6}=4.4 Hz, J_{4,5}=10.5 Hz, J_{5,6′}=10.3 Hz, 1H, H₅) and 3.45 (dd, J_{1′,2}=8.0 Hz, J_{1,1′}=13.9 Hz, 1H, H_{1′}); 101 MHz ¹³C NMR (CDCl₃) 165.1, 155.4, 137.0, 135.8, 133.6, 129.8, 129.3, 129.2, 128.7, 128.6, 128.4, 128.3, 128.1, 126.2, 101.6, 92.0 (d, J_{C3,F}=187.9 Hz, C₃), 78.1 (d, J=19.8 Hz), 70.5 (d, J=23.7 Hz), 69.6, 67.9, 52.5 (d, J_{C5,F}=7.3 HZ, C₅) and 45.1 (d, J_{Cl,F}=5.3 Hz, C₁) ppm and mass spectrum (m/e) 498 (M + Li).

### Example 18

### Preparation of N-Carbobenzoxy-4,6-O-benzylidene-1,3-dideoxy-3-fluoronojirimycin

To a solution of 1.92 g (3.9 mmol) of N-carbobenzoxy-2-O-benzoyl-4,6-O-benzylidene-1,3-dideoxy-3-fluoronojirimycin in 400 mL of dry methanol, 0.730 g (1.3 mmol) of sodium methoxide was added and the reaction stirred at room temperature under nitrogen atmosphere until the solution had cleared. An additional 500 mg of sodium methoxide was added before the reaction was complete by tlc (1% ethyl acetate, dichloromethane). The solution was neutralized with Dowex 50W-X8 resin (H form) and filtered immediately. The solvent was removed and the crude material was purified by silica gel chromatography using 1-3% ethyl acetate/methylene chloride as eluant to afford 1.10 g (73% yield) of the desired fluorohydrin; 400 MHz ¹H NMR (d, CDCl₃) 7.50-7.30 (m, 10H), 5.58 (s, 1H), 5.12 (AB quartet, 2H), 4.82 (ddd, J_{5,6}=4.5 Hz, J_{6,6′}=11.5 Hz, J_{6,F}=2.0 Hz, 1H, H₆), 4.45 (dt, J_{3,F}=52.3 Hz, J_{3,4}=J_{2,3}=8.3 Hz, 1H, H₃), 4.44 (t, J_{5,6′}=10.5 Hz, J_{6,6′}=11.5 Hz, 1H, H_{6′}), 4.30 (dt, J_{1,2}=5.0 Hz, J_{1,1′}=13.5 Hz, J_{1,F}=5.0 Hz, 1H, H₁) , 3.89 (dddd, J_{1,2}=5.0 Hz, J_{2,3}=8.3 Hz, J_{1′,2}=10.3 Hz, 1H, H₂), 3.87 (ddd, J_{3,4}=8.3 Hz, J_{4,5}=10.1 Hz, J_{4,F}=12.6 Hz, 1H, H₄), 3.30 (dt, J_{5,6}=4.6 Hz, J_{5,6′}=J_{4,5}=10.1 Hz, 1H, H₅) and 2.88 (dd, J_{1′,2}=10.2 Hz, J_{1,1′}=13.5 Hz, 1H, H_{1′}; 75 MHz ¹³C NMR (CDCl₃) 154.8, 137.0, 135.8, 129.3, 128.8, 128.6, 128.4, 128.3, 126.3, 101.4, 96.1 (d, J_{C3,F}=183 Hz) 78.2 (d, J=17.9 Hz); 69.2 (d, J=37 Hz) 68.3 (d, J=58 Hz) 54.25 (d, J=7.8 Hz) 48.4 (d, J=7.2 Hz).

### Example 19

### Preparation of 1,3-Dideoxy-3-fluoronojirimycin

To a solution of 1.00 g (2.5 mmol) of N-carbobenzoxy-4,6-O-benzylidene-1,3-dideoxy-3-fluoronojirimycin in 50 mL of glacial acetic acid was added 225 mg of 10% Pd/C and the solution was subjected to 50 psig H₂, with stirring, for 72 hours. The solution was filtered through celite and concentrated in vacuo, azeotroped with toluene, and dried on a vacuum pump to yield 478 mg of acetate salt. The acetate was removed by passage through a 10 ml column of amberlite CG400 (OH form) resin and eluted with 75 mL of water. The solution was lyopholized and recrystallized from ethanol/hexane to yield 379 mg (80% yield) of 1,3-dideoxy-3-fluoronojirimycin, mp 163°C; 500 MHz ¹H NMR (d, D₂O) 4.26 (dt, J_{3,F}=53.2 Hz, J_{2,3}=J_{3,4}=9.2 Hz, 1H, H₃), 3.80 (br d, J_{6,6′}=11.7 Hz, 1H, H₆), 3.76 (dddd, J_{1,2}=5.5 Hz, J_{1′,2}=11.9 Hz, J_{2,3}=9.0 Hz, J_{2,F}=5.3 Hz, 1H, H₂), 3.53 (dt, J_{3,4}=9.2 Hz, J_{4,5}=9.6 Hz, J_{4,F}=13.1 Hz, 1H, H₄), 3.14 (dt, J_{1,2}=5.4 Hz, J_{1,1′}=12.4 Hz, J_{1,F}=5.4 Hz, 1H, H₁), 2.60 (ddd, J_{5,6}=3.0 Hz, J_{5,6′}=5.5 Hz, J_{4,5}=9.6 Hz, 1H, H₅) and 2.51 (t, J_{1,2}=12.0 Hz, J_{1,1′}=12.4 Hz, 1H, H_{1′}); 75 MHz ¹³C NMR (D₂O) 102.3 (d, J_{C3,F}=179.5 Hz, C₃), 72.8 (d, J=16.8 Hz), 72.4 (d, J=16.5 Hz), 63.8, 62.9 (d, J_{C5,F}=6.6 Hz) and 50.7 (d, J_{Cl,F}=7.9 Hz, C₁) ppm; mass spectrum (m/e) 166 (M + H); and Anal. Calcd. for C₆H₁₂FNO₃: C (43.63), H (7.32) and N (8.48); Found C (43.85), H (7.41) and N (8.41).

### Example 20

### Preparation of N-Butyl-1,3-dideoxy-3-fluoronojirimycin

To a solution of 340 mg (2.06 mmol) of 1,3-dideoxy-3-fluoronojirimycin in 10 mL of methanol was added 297 mg (364 ml, 2 eq.) of n-butyraldehyde. The solution was placed in a hydrogenation bottle containing 250 mg of 10% Pd/C and subjected to a 5 psig of H₂ for 24 hours with stirring. An additional 2 equivalents of butyraldehyde was added and the solution stirred an additional 48 hours. The catalyst was removed by filtration, and the solvent removed in vacuo. This material was chromatographed on silica gel using 10% ethanol/methylene chloride as eluant and then recrystallized from ethanol/hexanes to afford 380 mg (85% yield) of N-butyl-1,3-dideoxy-3-fluoronojirimycin, mp 116°C; 500 MHz ¹H NMR (d, CD₃OD) 4.01 (dddd, J_{2,3}=8.9 Hz, J_{3,4}=9.2 Hz, J=1.0 Hz, J_{3,F}=53.6 Hz, 1H, H₃), 3.84 (AB quartet, J_{6,6′}=13.2 Hz, 2H, H₆ and H_{6′}), 3.68 (dddd, J_{1,2}=5.0 Hz, J_{1′,2}=11.0 Hz, J_{2,3}=8.9 Hz, J_{2,f}=4.2 Hz, 1H, H₂), 3.58 (dddd, J_{3,4}=9.2 Hz, J_{4,5}=9.4 Hz, J=1.0 Hz, J_{4,F}=14.0 Hz), 2.99 (dt, J_{1,2}=5.0 Hz, J_{1,1′}=11.0 HZ, J_{1,F}=5.0 Hz, 1H, H₁), 2.79 (dt, J=8.8 and 13.7 Hz, 1H), 2.56 (dt, J=7.8 and 13.7 Hz, 1H), 2.16 (t, J_{1′,2}=J_{1,1′}=11.0 Hz, 1H, H_{1′}), 2.11 (br d, J_{4,5}=9.4 Hz, 1H, H₅), 1.45 (m, 2H), 1.31, (m, 2H) and 0.93 (t, J=7.0 Hz, 3H); 75 MHz ¹³C NMR (CD₃oD) 100.8 (d, J_{C3,F}=181.4 Hz, C₃), 69.9 (d, J=20.1 Hz), 69.0 (d, J=17.5 Hz), 66.8 (d, J_{C5,F}=4.4 Hz, C₅), 58.8, 56.8 (d, J_{C1,F}=9.0 Hz, C₁), 53.2, 27.5, 21.7 and 14.3 ppm; mass spectrum (m/e) 222 (M + H) and 204; and Anal. Cald. for C₁₀H₂₀FNO₃: C (54.28), H (9.11) and N (6.33); Found C (54.21), H (9.14) and N (6.31).

### Example 21

### Preparation of N-Carbobenzoxy-2-O-acetyl-4,6-O-benzylidene-1-deoxynojirimycin

To a mixture of 0.50 g (1.30 mmol) of N-carbobenzoxy-4,6-O-enzylidene-1-deoxynojirimycin and 0.34 g (1.36 mmol, 1.05 eq) of di-n-butyltin oxide (both dried in vacuo over P₂O₅ overnight) under nitrogen, was added 5 mL of dry methanol. After refluxing for two hours, the solution was cooled, concentrated, toluene added and removed twice under vacuum to afford a white solid. This was dissolved in 5 mL of anhydrous methylene chloride under a nitrogen atmosphere and 0.20 mL (.15 g, 1.43 mmol, 1.10 eq) of dry triethyl-amine, followed by 92 ml (102 mg, 1.29 mmol, 1.0 eq) of acetyl chloride. After stirring at room temperature for one hour, 1N hydrochloric acid was added, the organic layer separated, dried with magnesium sulfate, filtered and concentrated under vacuum to afford 0.89 g of an oil, whose ¹H NMR spectrum indicated a 90:10 mixture of the 2-O-acetyl and 3-O-acetyl derivatives, respectively. Chromatography on a 2 mm silica gel chromatatron plate using methylene chloride, 1% methanol/methylene chloride, 2% methanol/methylene chloride and 5% methanol/methylene chloride afforded 0.24 g (44%) of N-=carbobenzoxy-2-O-acetyl-4,6-O-benzylidene-1-deoxynojirimycin as a white foam; 300 MHz ¹H NMR (d, CDCl₃) 7.52-7.30 (complex m, 1OH), 5.56 (s, 1H), 5.12 (AB quartet, J_{AB}=12.3 Hz, u_{AB}=19.8 Hz, 2H), 4.89-4.78 (complex m, 2H, H₂ and H₆), 4.23 (t, J_{6,6′}=10.7 Hz, 1H, H_{6′}), 4.12 (dd, J_{1,2}=4.4 Hz, H_{1,1′}=13.9 Hz, 1H, H₁), 3.79 (br t, J_{2,3}=J_{3,4}=9.7 Hz, 1H, H₃), 3.71 (t, J_{3,4}=J_{4,5}=9.7 Hz, 1H, H₄), 3.38 (ddd, J_{5,6}=4.4 Hz, J_{4,5}=J_{5,6′}=9.7 Hz, 1H, H₅), 3.17 (dd, J_{1′2}=8.1 Hz, J_{1,1′}=13.9 Hz, 1H, H_{1′}) and 2.80 (br s, 1H, OH); 75 MHz ¹³C NMR (CDCl₃), 170.4 (C), 155.2 (C), 137.1 (C), 136.0 (CO), 129.4 (CH), 128.7 (CH), 128.4 (CH), 128.3 (CH), 128.1 (CH), 126.3 (CH), 101.9 (CH), 80.1 (CH), 74.0 (CH), 69.4 (CH₂), 67.7 (CH₂), 53.5 (CH), 45.5 (CH₂) and 20.9 (CH₃) ppm; and mass spectrum (m/e) 434 (M + Li).

### Example 22

### Preparation of N-Carbobenzoxy-2-O-benzyl-4,6-O-benzylidene-1-deoxynojirimycin

To a mixture of 10.0 g (0.0260 mmol) of N-carbobenzoxy-4,6-O-benzylidene-1-deoxynojirimycin and 6.79 g (0.0273 mol) of dibutyl tin oxide, under nitrogen atmosphere, was added 100 mL of dry methanol and the mixture refluxed for three hours. After cooling to room temperature, the solvent was removed in vacuo. The crude foam was further dried by 2 x 100 mL toluene azeotropes to yield 16.0 g (99% yield) of N-carbobenzoxy-4,6-O-benzylidene-2,3-O-(di-n-butylstannylene)-1-deoxyojirimycin. The crude stannylene was dissolved in 65 mL (0.4 M) of acetonitrile and to this was added 2.0 g (20 mol %) of tetra-n-butylammonium iodide and 4.5 mL (1.5 eq) of benzylbromide and the mixture refluxed for 24 hours. After cooling, the solvents were removed in vacuo to yield 11.5 g (94%) of 3:1 mixture of 2-O-benzyl and 3-O-benzyl products, respectively. The mixture was chromatographed on a flash column (silica) using 0.5% methanol, 99.5% methylene chloride eluant to yield 4.5 g of pure N-carbobenzoxy-2-O-benzyl-4,6-O-benzylidene-1-deoxynojirimycin (38% yield), mp 112.5°C; 300 MHz ¹H NMR (d, CDCl₃), 7.51-7.27 (m, 15H), 5.53 (s, 1H), 5.10 (s, 2H), 4.78 (dd, H_{5,6}=4.5 Hz, H_{6,6′}=10.8 Hz, 1H, H₆), 4.67 (s, 2H), 4.28 (t, J_{5,6′}=J_{6,6′}=10.8 Hz, 1H, H_{6′},), 4.15 (dd, J_{1,1′}=13.5 Hz, J_{1′2}=4.1 Hz, 1H, H₁), 3.79 (dd, J_{2,3}=7.0 Hz, J_{3,4}=8.7 Hz, 1H, H₃), 3.64 (t, J_{4,5}=J_{3,4}=8.7 Hz, 1H, H₄), 3.48 (ddd, J_{1,2}=4.1 Hz, J_{1′,2}=9.1 Hz, J_{2,3}=7.0 Hz, 1H, H₂), 3.33 (ddd, J_{5,6}=4.5 Hz, J_{5,6}=10.3 Hz, J_{4,5}=8.7 Hz, 1H, H₅) and 3.01 (dd, J_{1,1′}=13.5 Hz, J_{1′.2}=9.1 Hz, 1H, H_{1′}); 75 MHz ¹³C NMR (CDCl₃) 155.1, 137.9, 137.3, 136.1, 129.4, 129.4, 129.3, 129.3, 129.2, 128.7, 128.6, 128.7, 128.4, 128.3, 128.2, 127.9, 127.8, 127.8, 126.3, 101.8, 80.4, 75.8, 72.3, 69.6, 67.6, 54.0 and 46.5; and mass spectrum (m/e) 482 (M + Li).

### Example 23

This example illustrates glycosidase inhibition activity for 1,2-dideoxy 2-fluoronojirimycin (1), N-butyl-1,2-dideoxy-2-fluoronojirimycin (2), 1,3-dideoxy-3-fluoronojirimycin (3), and N-butyl-1,3-dideoxy-3-fluoronojirimycin (4). It is contemplated that other N-derivatives will also manifest glycosidase inhibition activity.

The glycosidase inhibition activity is determined by modifying an assay procedure described in Evans et al, Phytochemistry, 22, pp. 768-770 (1983). More particularly, yeast α-glucosidase and almond β-glucosidase activities were measured by the Evans et al method which was modified by assaying activities at pH 7.4 in N-2-hydroxyethylpiperazine-N-2-ethane sulfonic acid (HEPES) buffer, measuring in 96 well microtiter plates, and including 10% DMSO in control and test samples.

The release of p-nitrophenol from the substrate p-nitrophenylglycoside was measured spectrophotometrically in the presence and absence of test compound. Each assay included a known inhibitor of the enzyme as a standard. IC₅₀ values were determined for compounds which inhibited the enzymes more than 50% at a 1 millimolar concentration.

### α-Glucosidase Inhibition Assay,

### pH 7.4

To 100 ul 50 mM HEPES buffer, pH 7.4, in a microtiter plate, 20 ul test compound in DMSO (DMSO alone in control)and 40 ul (0.013 units) yeast α-glucosidase (Sigma) in HEPES buffer were added and pre-incubated at room temperature 15 minutes. 40 µl 1.25 mM p-nitrophenyl-α-D-glucopyranoside (Sigma) in HEPES buffer, as substrate was added and the absorbance change at 405 nm was monitored in a Biotek EIA Autoreader. Absorption change was measured at 15 to 25 minutes (reaction was linear for at least 30 minutes). Each sample was tested in triplicate. IC₅₀ values were determined from the linear portion of the log concentration vs percent inhibition curve obtained from a minimum of 3 points. Deoxynojirimycin was used as standard inhibitor.

### β-Glucosidase Inhibition Assay

### pH 7.4:

To 100 µl 50 mM HEPES buffer, pH 7.4, in a microtiter plate, 20 µl test compound in DMSO (DMSO alone in control) and 40 ul (.136 units) β-glucosidase (Sigma) in HEPES buffer were added and pre-incubated at room temperature 15 minutes. 40 ul 1.25 mM p-nitrophenyl-β-D-glucopyranoside in HEPES buffer was added as substrate and the absorbance change at 405 nm was monitored utilizing a Biotek EIA Autoreader. Absorption change was measured at 15 to 25 minutes (reaction is linear for at least 30 minutes). Each sample was tested in triplicate. IC₅₀ values were determined from the linear portion of the log concentration vs percent inhibition curve obtained from a minimum of 3 points. Castanospermine was used as standard inhibitor.

### pH 4.8:

To 100 ul 50 mM sodium citrate buffer, pH 4.8, in a microtiter plate, 20 ul test compound in DMSO (DMSO alone in control) and 20 ul (.017 units) β-glucosidase (Sigma) in citrate buffer were added and pre-incubated at room temperature 15 minutes. 20 ul 2.50 mM p-nitrophenyl-β-D-glucopyranside in citrate buffer was added as substrate and incubated at room temperature 20 minutes (reaction is linear for at least 30 minutes). 50 ul 0.4 M NaOH was added and the absorption change at 405 nm was determined utilizing a Biotek EIA Autoreader. Each sample was tested in triplicate. IC₅₀ values were determined from the linear portion of the log concentration vs percent inhibition curve obtained from a minimum of 3 points. Castanospermine was used as standard inhibitor.

**Table 1**

| Enzyme and Virus Inhibition Data | | | | | |
|---|---|---|---|---|---|
| COMPOUND NO. | ALPHA GLUCOSIDASE | BETA GLUCOSIDASE-pH4.8 | BETA GLUCOSIDASE-pH7.4 | ALPHA MANNOSIDASE-pH4.5 | ALPHA MANNOSIDASE-pH7.4 |
| 1 | 24% @ 1mM | 6% @ 1mM | 11% @ 1mM | 4% @ 1mM | 19% @ 1mM |
| | 64% @ 5mM | 25% @ 5mM | 25% @ 5mM | 5% @ 5mM | 11% @ 5mM |
| 2 | 8% @ 1mM | 2% @ 1mM | 4% @ 1mM | 3% @ 1mM | 4% @ 1mM |
| | 13% @ 5mM | -6% @ 5mM | 11% @ 5mM | 5% @ 5mM | 12% @ 5mM |
| 3 | 26% @ 1mM | 4% @ 1mM | 3% @ 1mM | 11% @ 1mM | 10% @ 1mM |
| | 63% @ 5mM | 10% @ 5mM | 24% @ 5mM | 19% @ 5mM | 30% @ 5mM |
| 4 | 11% @ 1mM | 8% @ 1mM | 0% @ 1mM | 6% @ 1mM | 7% @ 1mM |
| | 18% @ 5mM | -10% @ 5mM | 2% @ 5mM | 5% @ 5mM | 11% @ 5mM |

The preceding examples can be repeated with similar success by substituting the generically or specifically described reactants and/or operating conditions of this invention for those used in the preceding examples.

## Claims

1. Compounds represented by the formulas: 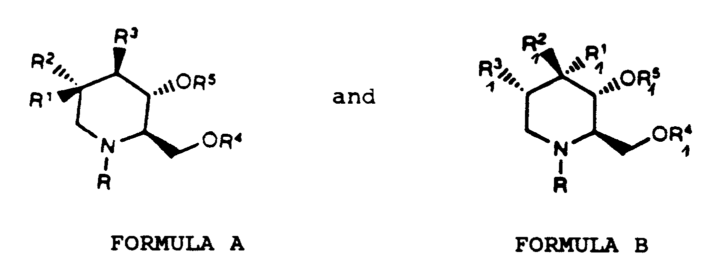 wherein R represents hydrogen, alkyl radicals having from 1 to 10 carbon atoms, alkenyl radicals having to 10 carbon atoms, aryl, alkaryl and aralkyl radicals having from 6 to 16 carbon atoms and acyl and acyloxy radicals of the formula 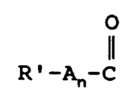 wherein R' represents alkyl radicals having from 1 to 10 carbon atoms, and aryl, aralkyl and alkaryl radicals having from 6 to 26 carbon atoms; A represents oxygen; n is 0 or 1; R¹ represents hydrogen; R² represents hydrogen, fluorine and sulfonyl esters represented by the formula: 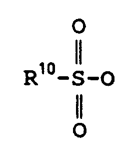 wherein R¹⁰ represents alkyl radicals having from 1 to 6 carbon atoms and aryl, aralkyl and alkaryl radicals, R³ represents hydroxy or together with R² represents a cyclic stannylene derivative of the formula: 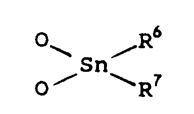 wherein R⁶ and R⁷ independently represent alkyl radicals having from 1 to 10 carbon atoms, or R³ together with R¹ represents an epoxide; provided that when R² is fluorine, R³ is hydroxy and when R² is hydrogen, R¹ and R³ together form an epoxide; R⁴ and R⁵ represent hydrogen and hydroxy protecting groups wherein R⁴ and R⁵ together represent a group of the formula 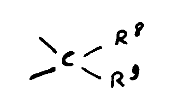 wherein R₈ and R₉ independently represent hydrogen, alkyl radicals having from 1 to 10 carbon atoms and aryl radicals;
R₁¹ represents hydrogen and hydroxy; R₁² represents hydrogen, hydroxy and fluorine or together with R₁¹ represents a keto group; R₁³ represents hydroxy, benzyl and allyl ethers, and acyl esters represented by the formula: 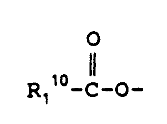 wherein R₁¹⁰ represents alkyl radicals having from 1 to 10 carbon atoms,aryl, aralkyl and alkaryl radicals, or together with R₁² represents a cyclic stannylene derivative of the formula: 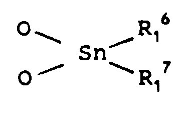 wherein R₁⁶ and R₁⁷ independently represent alkyl radicals having from 1 to 10 carbon atoms; provided that when R₁² is fluorine, R₁³ is hydroxy, an acyl ester, benzyl ether or allyl ether, and when R₁¹ is hydroxy, R₁³ is hydroxy, an acyl ester or a benzyl or allyl ether, further provided that when R₁² is hydroxy, then R₁³ is not hydroxy, further provided that when R₁¹ is hydroxy, R₁² is hydrogen and further provided that only one of R₁¹ and R₁² may concurrently be hydrogen; and R₁⁴ and R₁⁵ represent hydroxy protecting groups wherein R₁⁴ and R₁⁵ together represent a group of the formula 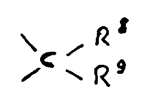 wherein R⁸ and R⁹ have the same meaning as defined above,
with the proviso that the formula A and B do not include the formula C and D 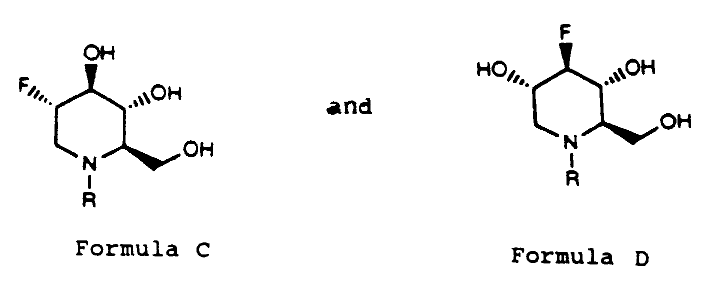 wherein R represents hydrogen, alkyl radicals having from 1 to 10 carbon atoms, alkenyl radicals having to 10 carbon atoms, aryl, alkaryl and aralkyl radicals having from 6 to 16 carbon atoms and acyl and acyloxy radicals of the formula 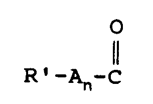 wherein R' represents alkyl radicals having from 1 to 10 carbon atoms, and aryl, aralkyl and alkaryl radicals having from 6 to 26 carbon atoms; A represents oxygen; n is 0 or 1.

2. Compounds represented by the formula: 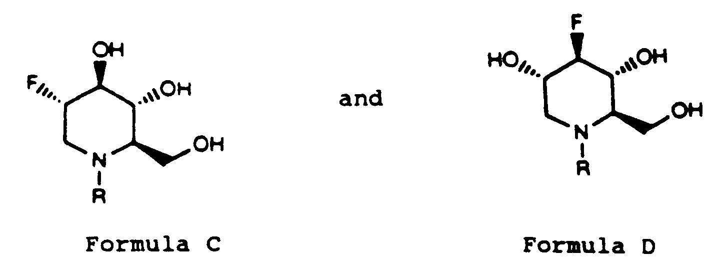 wherein R represents hydrogen, alkyl radicals having from 1 to 10 carbon atoms, alkenyl radicals having to 10 carbon atoms, aryl, alkaryl and aralkyl radicals having from 6 to 16 carbon atoms and acyl and acyloxy radicals of the formula 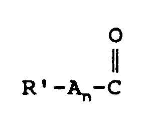 wherein R' represents alkyl radicals having from 1 to 10 carbon atoms, and aryl, aralkyl and alkaryl radicals having from 6 to 26 carbon atoms; A represents oxygen; n is 0 or 1.

3. Compounds of Claim 2 wherein R represents hydrogen.

4. Compounds of Claim 2 wherein R represents an alkyl radical having from 1 to 10 carbon atoms.

5. Compounds of Claim 2 wherein R represents an alkyl radical having from 1 to 6 carbon atoms.

6. Compounds of Claim 2 wherein R represents an alkyl radical having 4 carbon atoms.

7. Compounds of Claim 2 wherein R is n-butyl.

8. Compounds of Claim 1 represented by the formulas: 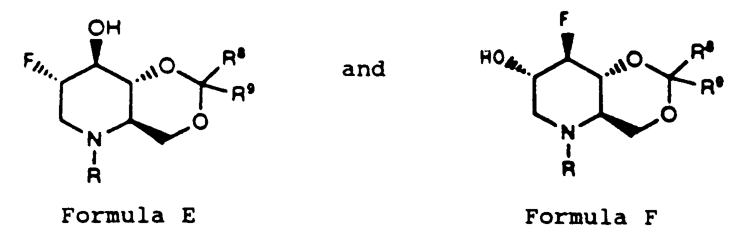 wherein R represents hydrogen, alkyl radicals having from 1 to 10 carbon atoms, alkenyl radicals having to 10 carbon atoms, aryl, aralkyl and alkaryl radicals having from 6 to 16 carbon atoms and acyl and acyloxy radicals as defined in claim 1,
and R⁸ and R⁹ independently represent hydrogen, alkyl radicals having from 1 to 10 carbon atoms and aryl radicals.

9. Compounds of Claim 8 wherein R represents hydrogen.

10. Compounds of Claim 8 wherein R represents an alkyl radical having from 1 to 10 carbon atoms.

11. Compounds of Claim 8 wherein R represents an alkyl radical having from 1 to 6 carbon atoms.

12. Compounds of Claim 8 wherein R represents an alkyl radical having 4 carbon atoms.

13. Compounds of Claim 8 wherein R is n-butyl.

14. Compounds of Claim 8 wherein R represents a carbobenzoxy radical.

15. Compounds of Claim 8 wherein R represents a butyryl radical.

16. Compounds of Claim 1 represented by the formulas: 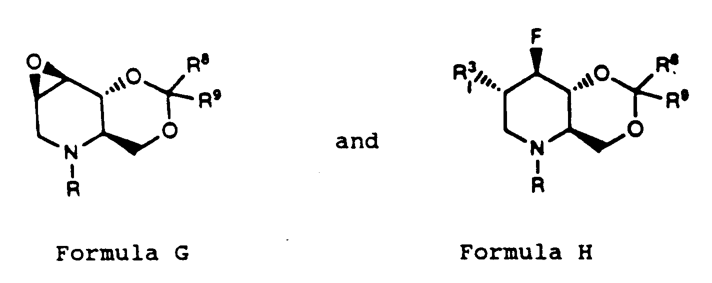 wherein R represents hydrogen, alkyl radicals having from 1 to 10 carbon atoms, alkenyl radicals having to 10 carbon atoms, substituted aryl, alkaryl and aralkyl radicals having from 7 to 14 carbon atoms in the case of Formula G and from 6 to 16 carbon atoms in the case of Formula H and acyl and acyloxy radicals as defined in claim 1; R₁³ represents benzyl and allyl ethers, and acyl esters represented by the formula: 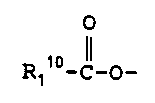 wherein R₁¹⁰ represents alkyl radicals having from 1 to 10 carbon atoms and aryl, aralkyl and alkaryl radicals; and R⁸ and R⁹ independently represent hydrogen, alkyl radicals having from 1 to 10 carbon atoms and aryl radicals.

17. Compounds of Claim 16 wherein R represents hydrogen.

18. Compounds of Claim 16 wherein R represents an alkyl radical having from 1 to 10 carbon atoms.

19. Compounds of Claim 16 wherein R represents an alkyl radical having from 1 to 6 carbon atoms.

20. Compounds of Claim 16 wherein R represents an alkyl radical having 4 carbon atoms.

21. Compounds of Claim 16 wherein R is n-butyl.

22. Compounds of Claim 16 wherein R represents a carbobenzoxy radical.

23. Compounds of Claim 16 wherein R represents a butyryl radical.

24. Compounds of Claim 1 represented by the formulas: 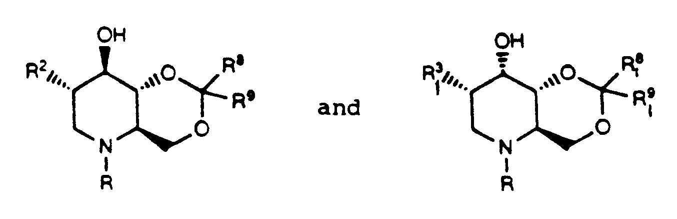 wherein R represents hydrogen, alkyl radicals having from 1 to 10 carbon atoms, alkenyl radicals having to 10 carbon atoms, aryl, alkaryl and aralkyl radicals having from 6 to 16 carbon atoms and acyl and acyloxy as defined in claim 1; R² represents a sulfonyl ester represented by the formula: 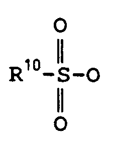 wherein R¹⁰ represents alkyl radicals having from 1 to 6 carbon atoms and aryl, aralkyl and alkaryl radicals; and R₁³ represents a benzyl or allyl ether or an acyl ester represented by the formula: 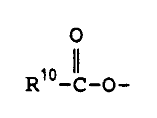 wherein R¹⁰ represents alkyl radicals having from 1 to 10 carbon atoms and aryl, aralkyl and alkaryl radicals; and R⁸ and R⁹ independently represent hydrogen, alkyl radicals having from 1 to 10 carbon atoms and aryl radicals.

25. Compounds of Claim 24 wherein R represents hydrogen.

26. Compounds of Claim 24 wherein R represents an alkyl radical having from 1 to 10 carbon atoms.

27. Compounds of Claim 24 wherein R represents an alkyl radical having from 1 to 6 carbon atoms.

28. Compounds of Claim 24 wherein R represents an alkyl radical having 4 carbon atoms.

29. Compounds of Claim 24 wherein R is n-butyl.

30. Compounds of Claim 24 wherein R represents a carbobenzoxy radical.

31. Compounds of Claim 24 wherein R represents a butyryl radical.

32. Compounds of Claim 1 represented by the formulas: 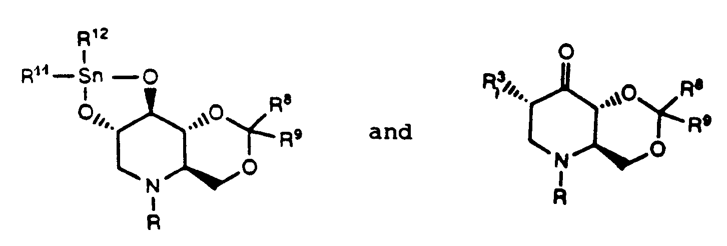 wherein R represents hydrogen, alkyl radicals having from 1 to 10 carbon atoms, alkenyl radicals having to 10 carbon atoms, aryl, alkaryl and aralkyl radicals having from 6 to 16 carbon atoms and acyl and acyloxy radicals as defined in claim 1; R₁³ represents a benzyl or allyl ether or an acyl ester represented by the formula: 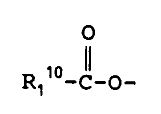 wherein R₁¹⁰ represents alkyl radicals having from 1 to 10 carbon atoms and aryl, aralkyl and alkaryl radicals, R⁸ and R⁹ independently represent hydrogen, alkyl radicals having from 1 to 10 carbon atoms and aryl radicals, and R¹¹ and R¹² represent alkyl radicals having from 1 to 10 carbon atoms.

33. Compounds of Claim 32 wherein R represents hydrogen.

34. Compounds of Claim 32 wherein R represents an alkyl radicals having from 1 to 10 carbon atoms.

35. Compounds of Claim 32 wherein R represents an alkyl radical having from 1 to 6 carbon atoms.

36. Compounds of Claim 32 wherein R represents an alkyl radical having 4 carbon atoms.

37. Compounds of Claim 32 wherein R is n-butyl.

38. Compounds of Claim 32 wherein R represents a carbobenzoxy radical.

39. Compounds of Claim 32 wherein R represents a butyryl radical.

40. Compound of Claim 1 represented by the formula: 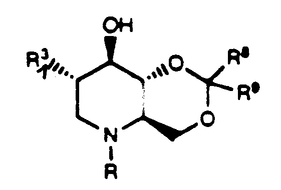 wherein R represents hydrogen, alkyl radicals having from 1 to 10 carbon atoms, alkenyl radicals having to 10 carbon atoms, aryl, alkaryl and aralkyl radicals having from 6 to 16 carbon atoms and acyl and acyloxy as defined in claim 1; R₁³ represents a benzyl or allyl ether or an acyl ester of the formula: 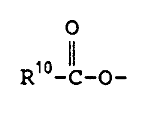 wherein R¹⁰ represents alkyl radicals having from 1 to 10 carbon atoms and aryl, aralkyl and alkaryl radicals; and R⁸ and R⁹ independently represent hydrogen, alkyl radicals having from 1 to 10 carbon atoms and aryl radicals.

41. Compound of Claim 40 wherein R represents hydrogen.

42. Compound of Claim 40 wherein R represents an alkyl radical having from 1 to 10 carbon atoms.

43. Compound of Claim 40 wherein R represents an alkyl radical having from 1 to 6 carbon atoms.

44. Compound of Claim 40 wherein R represents an alkyl radical having 4 carbon atoms.

45. Compound of Claim 40 wherein R is n-butyl.

46. Compound of Claim 40 wherein R represent a carbobenzoxy radical.

47. Compound of Claim 40 wherein R represents a butyryl radical.

48. Composition comprising a compound of Claim 2.

49. N-butyl-1,2-dideoxy-2-fluoronojirimycin.

50. 1,2-dideoxy-2-fluoronojirimycin.

51. N-butyl-1,3-dideoxy-3-fluoronojirimycin.

52. 1,3-dideoxy-3-fluoronojirimycin.

## Patentansprüche

1. Verbindungen der Formeln: 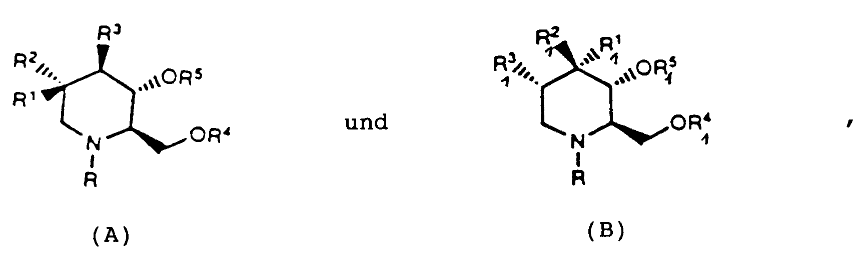 worin R bedeutet: Wasserstoff, Alkylreste mit 1 bis 10 Kohlenstoffatomen, Alkenylreste mit bis zu 10 Kohlenstoffatomen, Aryl-, Alkaryl- und Aralkylreste mit 6 bis 16 Kohlenstoffatomen, und Acyl- und Acyloxyreste der Formel 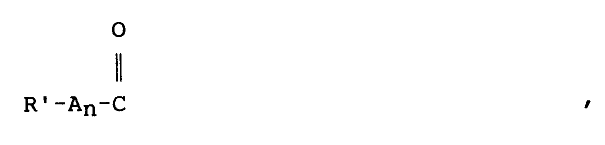 worin R' darstellt: Alkylreste mit 1 bis 10 Kohlenstoffatomen, und Aryl-, Aralkyl- und Alkarylreste mit 6 bis 26 Kohlenstoffatomen; A Sauerstoff bedeutet; n Null oder 1 ist; R¹ Wasserstoff darstellt; R² bedeutet: Wasserstoff, Fluor und Sulfonylester der Formel: 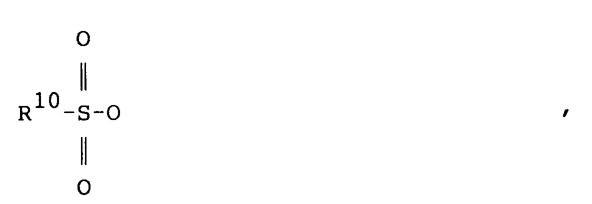 worin R¹⁰ darstellt: Alkylreste mit 1 bis 6 Kohlenstoffatomen, und Aryl-, Aralkyl- und Alkarylreste; R³ Hydroxy ist, oder zusammen mit R² bedeutet: ein cyclisches Stannylen-Derivat der Formel 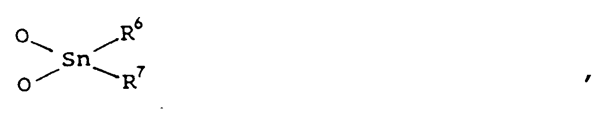 worin R⁶ und R⁷ unabhängig Alkylreste mit 1 bis 10 Kohlenstoffatomen darstellen, oder R³ zusammen mit R¹ ein Epoxid ist; mit der Maßgabe, daß, wenn R² Fluor bedeutet, R³ Hydroxy darstellt, und wenn R² Wasserstoff ist, R¹ und R² zusammen ein Epoxid bilden; R⁴ und R⁵ bedeuten: Wasserstoff und Hydroxy-Schutzgruppen, worin R⁴ und R⁵ zusammen eine Gruppe darstellen der Formel: 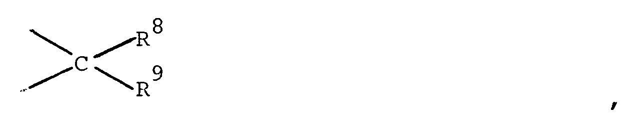 worin R⁸ und R⁹ unabhängig Wasserstoff, Alkylreste mit 1 bis 10 Kohlenstoffatomen und Arylreste sind; R₁¹ Wasserstoff und Hydroxy bedeutet; R₁² Wasserstoff, Hydroxy und Fluor ist, oder zusammen mit R₁¹ eine Keto-Gruppe ist; R₁³ darstellt: Hydroxy, Benzyl- und Allylether, und Acylester der Formel: 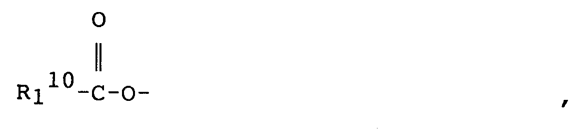 worin R₁¹⁰ Alkylreste mit 1 bis 10 Kohlenstoffatomen, Aryl-, Aralkyl- und Alkarylreste bedeutet, oder zusammen mit R₁² darstellt: ein cyclisches Stannylen-Derivat der Formel: 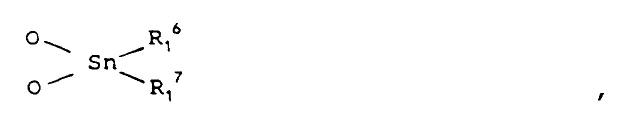 worin R₁⁶ und R₁⁷ unabhängig Alkylreste mit 1 bis 10 Kohlenstoffatomen bedeuten; mit der Maßgabe, daß, wenn R₁² Fluor ist, R₁³ Hydroxy, einen Acylester, Benzylether oder Allylether darstellt, und wenn R₁¹ Hydroxy bedeutet, R₁³ Hydroxy, ein Acylester oder ein Benzyl- oder Allylether ist; ferner mit der Maßgabe, daß, wenn R₁² Hydroxy darstellt, R₁³ nicht Hydroxy bedeutet; ferner mit der Maßgabe, daß, wenn R₁¹ Hydroxy darstellt, R₁² Wasserstoff ist; und ferner mit der Maßgabe, daß nur eines von R₁¹ und R₁² gleichzeitig Wasserstoff sein kann; R₁⁴ und R₁⁵ Hydroxy-Schutzgruppen darstellen, wobei R₁⁴ und R₁⁵ gemeinsam darstellen: eine Gruppe der Formel 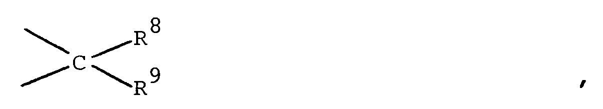 worin R⁸ und R⁹ dieselbe Bedeutung wie oben definiert haben, mit der Maßgabe, daß die Formeln (A) und (B) nicht einschließen die Formeln (C) und (D): 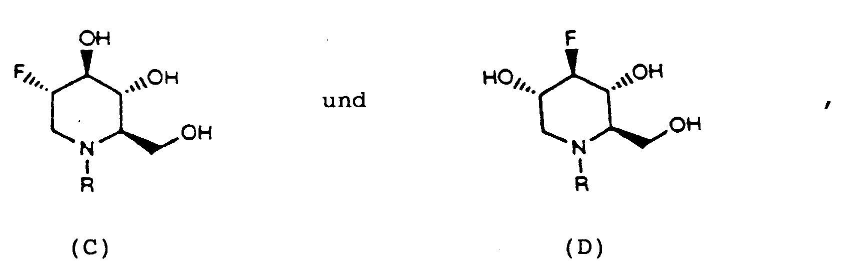 worin R bedeutet: Wasserstoff, Alkylreste mit 1 bis 10 Kohlenstoffatomen, Alkenylreste mit bis zu 10 Kohlenstoffatomen, Aryl-, Alkaryl- und Aralkylreste mit 6 bis 16 Kohlenstoffatomen, und Acyl- und Acyloxyreste der Formel 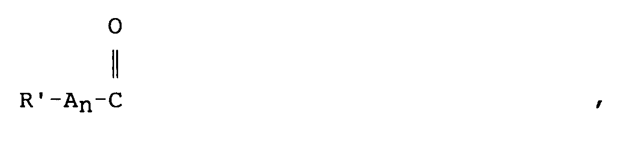 worin R' darstellt: Alkylreste mit 1 bis 10 Kohlenstoffatomen, und Aryl-, Aralkyl- und Alkarylreste mit 6 bis 26 Kohlenstoffatomen; A Sauerstoff bedeutet; und n Null oder 1 ist.

2. Verbindungen der Formeln: 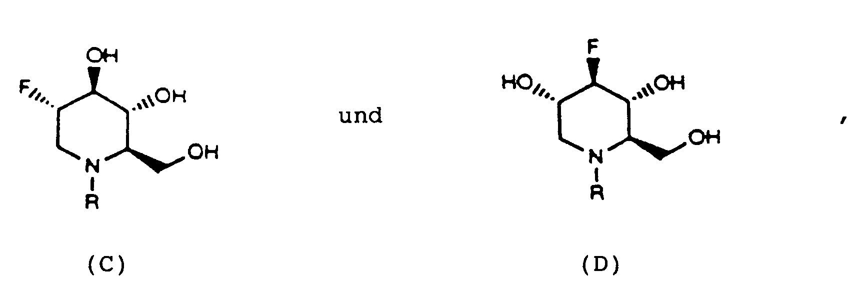 worin R bedeutet: Wasserstoff, Alkylreste mit 1 bis 10 Kohlenstoffatomen, Alkenylreste mit bis zu 10 Kohlenstoffatomen, Aryl-, Alkaryl- und Aralkylreste mit 6 bis 16 Kohlenstoffatomen und Acyl- und Acyloxyreste der Formel 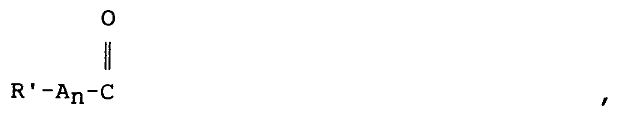 worin R' darstellt: Alkylreste mit 1 bis 10 Kohlenstoffatomen, und Aryl-, Aralkyl- und Alkarylreste mit 6 bis 26 Kohlenstoffatomen; A Sauerstoff bedeutet; und n Null oder 1 ist.

3. Verbindungen nach Anspruch 2, worin R Wasserstoff bedeutet.

4. Verbindungen nach Anspruch 2, worin R einen Alkylrest mit 1 bis 10 Kohlenstoffatomen darstellt.

5. Verbindungen nach Anspruch 2, worin R einen Alkylrest mit 1 bis 6 Kohlenstoffatomen darstellt.

6. Verbindungen nach Anspruch 2, worin R einen Alkylrest mit 4 Kohlenstoffatomen darstellt.

7. Verbindungen nach Anspruch 2, worin R n-Butyl ist.

8. Verbindungen nach Anspruch 1 der Formeln: 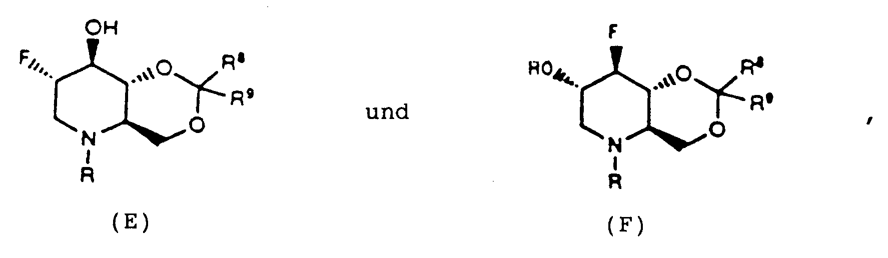 worin R bedeutet: Wasserstoff, Alkylreste mit 1 bis 10 Kohlenstoffatomen, Alkenylreste mit bis zu 10 Kohlenstoffatomen, Aryl-, Aralkyl- und Alkarylreste mit 6 bis 16 Kohlenstoffatomen, und Acyl- und Acyloxyreste, wie in Anspruch 1 definiert; und R⁸ und R⁹ unabhängig Wasserstoff, Alkylreste mit 1 bis 10 Kohlenstoffatomen und Arylreste darstellen.

9. Verbindungen nach Anspruch 8, worin R Wasserstoff bedeutet.

10. Verbindungen nach Anspruch 8, worin R einen Alkylrest mit 1 bis 10 Kohlenstoffatomen darstellt.

11. Verbindungen nach Anspruch 8, worin R einen Alkylrest mit 1 bis 6 Kohlenstoffatomen darstellt.

12. Verbindungen nach Anspruch 8, worin R einen Alkylrest mit 4 Kohlenstoffatomen darstellt.

13. Verbindungen nach Anspruch 8, worin R n-Butyl ist.

14. Verbindungen nach Anspruch 8, worin R einen Carbobenzoxyrest darstellt.

15. Verbindungen nach Anspruch 8, worin R einen Butyrylrest darstellt.

16. Verbindungen nach Anspruch 1 der Formeln: 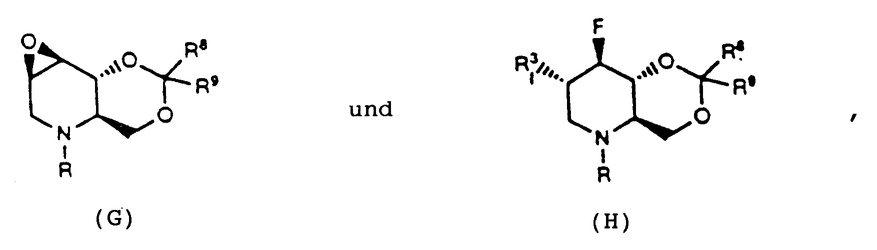 worin R bedeutet: Wasserstoff, Alkylreste mit 1 bis 10 Kohlenstoffatomen, Alkenylreste mit bis zu 10 Kohlenstoffatomen, substituierte Aryl-, Alkaryl- und Aralkylreste mit 7 bis 14 Kohlenstoffatomen im Fall von Formel (G) und 6 bis 16 Kohlenstoffatomen im Fall von Formel (H), und Acyl- und Acyloxyreste, wie in Anspruch 1 definiert; R₁³ darstellt: Benzyl- und Allylether, und Acylester der Formel: 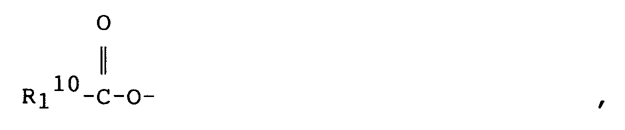 worin R₁¹⁰ Alkylreste mit 1 bis 10 Kohlenstoffatomen, und Aryl-, Aralkyl- und Alkarylreste bedeutet; und R⁸ und R⁹ unabhängig Wasserstoff, Alkylreste mit 1 bis 10 Kohlenstoffatomen und Arylreste darstellen.

17. Verbindungen nach Anspruch 16, worin R Wasserstoff bedeutet.

18. Verbindungen nach Anspruch 16, worin R einen Alkylrest mit 1 bis 10 Kohlenstoffatomen darstellt.

19. Verbindungen nach Anspruch 16, worin R einen Alkylrest mit 1 bis 6 Kohlenstoffatomen darstellt.

20. Verbindungen nach Anspruch 16, worin R einen Alkylrest mit 4 Kohlenstoffatomen darstellt.

21. Verbindungen nach Anspruch 16, worin R n-Butyl ist.

22. Verbindungen nach Anspruch 16, worin R einen Carbobenzoxyrest darstellt.

23. Verbindungen nach Anspruch 16, worin R einen Butyrylrest darstellt.

24. Verbindungen nach Anspruch 1 der Formeln: 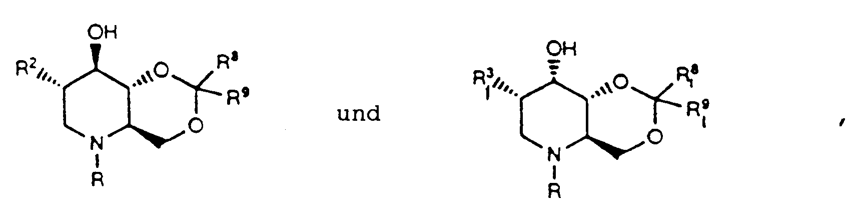 worin R bedeutet: Wasserstoff, Alkylreste mit 1 bis 10 Kohlenstoffatomen, Alkenylreste mit bis zu 10 Kohlenstoffatomen, Aryl-, Alkaryl- und Aralkylreste mit 6 bis 16 Kohlenstoffatomen, und Acyl- und Acyloxyreste, wie in Anspruch 1 definiert; R² bedeutet: einen Sulfonylester der Formel: 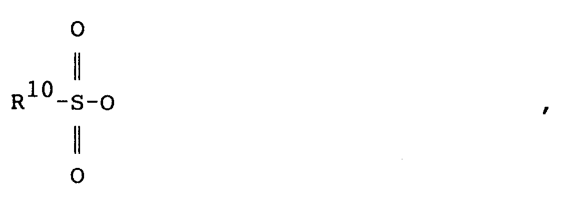 worin R¹⁰ darstellt: Alkylreste mit 1 bis 6 Kohlenstoffatomen, und Aryl-, Aralkyl- und Alkarylreste; R₁³ bedeutet: einen Benzyl- oder Allylether, oder einen Acylester der Formel: 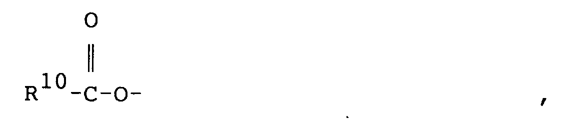 worin R¹⁰ Alkylreste mit 1 bis 10 Kohlenstoffatomen, und Aryl-, Aralkyl- und Alkarylreste bedeutet; und R⁸ und R⁹ unabhängig Wasserstoff, Alkylreste mit 1 bis 10 Kohlenstoffatomen und Arylreste darstellen.

25. Verbindungen nach Anspruch 24, worin R Wasserstoff bedeutet.

26. Verbindungen nach Anspruch 24, worin R einen Alkylrest mit 1 bis 10 Kohlenstoffatomen darstellt.

27. Verbindungen nach Anspruch 24, worin R einen Alkylrest mit 1 bis 6 Kohlenstoffatomen darstellt.

28. Verbindungen nach Anspruch 24, worin R einen Alkylrest mit 4 Kohlenstoffatomen darstellt.

29. Verbindungen nach Anspruch 24, worin R n-Butyl ist.

30. Verbindungen nach Anspruch 24, worin R einen Carbobenzoxyrest darstellt.

31. Verbindungen nach Anspruch 24, worin R einen Butyrylrest darstellt.

32. Verbindungen nach Anspruch 1 der Formeln: 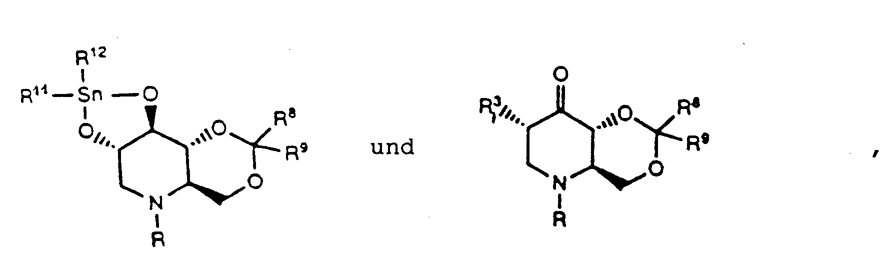 worin R bedeutet: Wasserstoff, Alkylreste mit 1 bis 10 Kohlenstoffatomen, Alkenylreste mit bis zu 10 Kohlenstoffatomen, Aryl-, Alkaryl- und Aralkylreste mit 6 bis 16 Kohlenstoffatomen, und Acyl- und Acyloxyreste, wie in Anspruch 1 definiert; R₁³ darstellt: einen Benzyl- oder Allylether, oder einen Acylester der Formel: 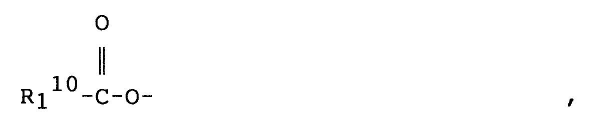 worin R₁¹⁰ Alkylreste mit 1 bis 10 Kohlenstoffatomen, und Aryl-, Aralkyl- und Alkarylreste bedeutet; R⁸ und R⁹ unabhängig Wasserstoff, Alkylreste mit 1 bis 10 Kohlenstoffatomen und Arylreste darstellen; und R¹¹ und R¹² Alkylreste mit 1 bis 10 Kohlenstoffatomen sind.

33. Verbindungen nach Anspruch 32, worin R Wasserstoff bedeutet.

34. Verbindungen nach Anspruch 32, worin R einen Alkylrest mit 1 bis 10 Kohlenstoffatomen darstellt.

35. Verbindungen nach Anspruch 32, worin R einen Alkylrest mit 1 bis 6 Kohlenstoffatomen darstellt.

36. Verbindungen nach Anspruch 32, worin R einen Alkylrest mit 4 Kohlenstoffatomen darstellt.

37. Verbindungen nach Anspruch 32, worin R n-Butyl ist.

38. Verbindungen nach Anspruch 32, worin R einen Carbobenzoxyrest darstellt.

39. Verbindungen nach Anspruch 32, worin R einen Butyrylrest darstellt.

40. Verbindung nach Anspruch 1 der Formel: 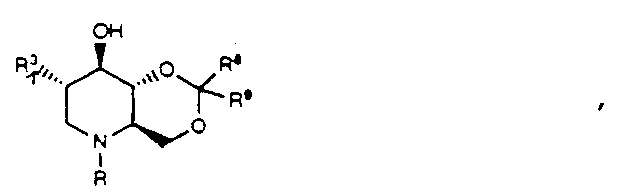 worin R bedeutet: Wasserstoff, Alkylreste mit 1 bis 10 Kohlenstoffatomen, Alkenylreste mit bis zu 10 Kohlenstoffatomen, Aryl-, Alkaryl- und Aralkylreste mit 6 bis 16 Kohlenstoffatomen und Acyl und Acyloxyreste, wie in Anspruch 1 definiert; R₁³ darstellt: einen Benzyl- oder Allylether oder einen Acylester der Formel: 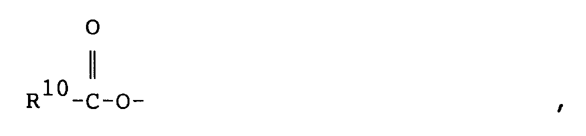 worin R¹⁰ Alkylreste mit 1 bis 10 Kohlenstoffatomen, Aryl-, Aralkyl- und Alkarylreste bedeutet; und R⁸ und R⁹ unabhängig Wasserstoff, Alkylreste mit 1 bis 10 Kohlenstoffatomen und Arylreste darstellen.

41. Verbindungen nach Anspruch 40, worin R Wasserstoff bedeutet.

42. Verbindungen nach Anspruch 40, worin R einen Alkylrest mit 1 bis 10 Kohlenstoffatomen darstellt.

43. Verbindungen nach Anspruch 40, worin R einen Alkylrest mit 1 bis 6 Kohlenstoffatomen darstellt.

44. Verbindungen nach Anspruch 40, worin R einen Alkylrest mit 4 Kohlenstoffatomen darstellt.

45. Verbindungen nach Anspruch 40, worin R n-Butyl ist.

46. Verbindungen nach Anspruch 40, worin R einen Carbobenzoxyrest darstellt.

47. Verbindungen nach Anspruch 40, worin R einen Butyrylrest darstellt.

48. Zusammensetzung, welche eine Verbindung nach Anspruch 2 umfaßt.

49. N-Butyl-1,2-didesoxy-2-fluornojirimycin.

50. 1,2-Didesoxy-2-fluornojirimycin.

51. N-Butyl-1,3-didesoxy-3-fluornojirimycin.

52. 1,3-Didesoxy-3-fluornojirimycin.

## Revendications

1. Composés représentés par les formules : 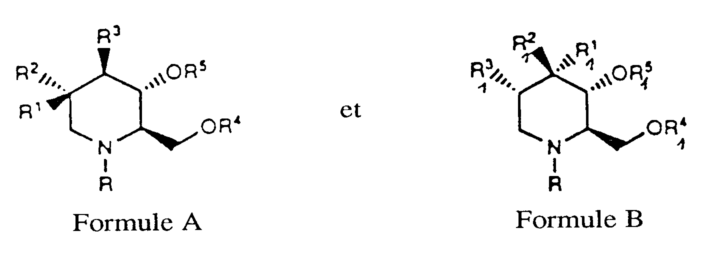 dans lesquelles
les R représentent des atomes d'hydrogène, des groupes alkyle comportant de 1 à 10 atomes de carbone, des groupes alcényle comportant jusqu'à 10 atomes de carbone, des groupes aryle, alkaryle ou aralkyle comportant de 6 à 16 atomes de carbone, ou des groupes acyle ou acyloxy de formule 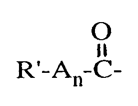 dans laquelle R' représente un groupe alkyle comportant de 1 à 10 atomes de carbone, ou un groupe aryle, alkaryle ou aralkyle comportant de 6 à 26 atomes de carbone, A représente un atome d'oxygène, et n vaut 0 ou 1;
R¹ représente un atome d'hydrogène;
R² représente un atome d'hydrogène ou de fluor ou un groupe ester sulfonique de formule 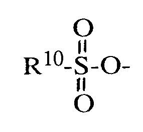 dans laquelle R¹⁰ représente un groupe alkyle comportant de 1 à 6 atomes de carbone, ou un groupe aryle, aralkyle ou alkaryle;
R³ représente un groupe hydroxy, ou bien, conjointement avec R², un groupe cyclique de type stannylène, de formule 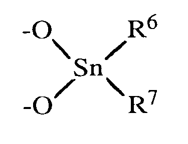 dans laquelle R⁶ et R⁷ représentent chacun, indépendamment, un groupe alkyle comportant de 1 à 10 atomes de carbone,
ou bien R³, conjointement avec R¹, représente un substituant époxy ;
sous réserve que, quand R² représente un atome de fluor, R³ représente un groupe hydroxy, et que, quand R² représente un atome d'hydrogène, R¹ et R³ constituent conjointement un substituant époxy ;
R⁴ et R⁵ représentent des atomes d'hydrogène ou des groupes hydroxy-protecteurs, où R⁴ et R⁵ constituent conjointement un groupe de formule 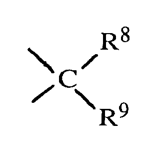 dans laquelle R⁸ et R⁹ représentent chacun, indépendamment, un atome d'hydrogène, un groupe alkyle comportant de 1 à 10 atomes de carbone ou un groupe aryle;
R₁¹ représente un atome d'hydrogène ou un groupe hydroxy;
R₁² représente un atome d'hydrogène ou de fluor ou un groupe hydroxy, ou bien, conjointement avec R₁¹, un substituant oxo ;
R₁³ représente un groupe hydroxy, benzyloxy ou allyloxy, ou encore un groupe acyloxy représenté par la formule 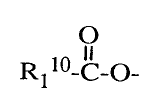 dans laquelle R₁¹⁰ représente un groupe alkyle comportant de 1 à 10 atomes de carbone, ou un groupe aryle, aralkyle ou alkaryle,
ou bien, conjointement avec R₁², un groupe cyclique de type stannylène, de formule 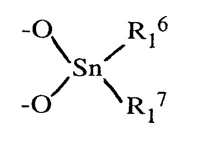 dans laquelle R₁⁶ et R₁⁷ représentent chacun, indépendamment, un groupe alkyle comportant de 1 à 10 atomes de carbone ;
sous réserve que, quand R₁² représente un atome de fluor, R₁³ représente un groupe hydroxy, acyloxy, benzyloxy ou allyloxy, et que, quand R₁¹ représente un groupe hydroxy, R₁³ représente un groupe hydroxy, acyloxy, benzyloxy ou allyloxy, et encore que, quand R₁² représente un groupe hydroxy, R₁³ ne représente pas un groupe hydroxy, et que, quand R₁¹ représente un groupe hydroxy, R₁² représente un atome d'hydrogène, et enfin, qu'un seul à la fois des symboles R₁¹ et R₁² représente un atome d'hydrogène; et
R₁⁴ et R₁⁵ représentent des groupes hydroxy-protecteurs, où R₁⁴ et R₁⁵ constituent conjointement un groupe de formule 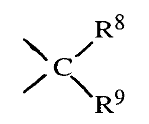 dans laquelle R⁸ et R⁹ ont les mêmes significations que celles indiquées plus haut;
sous réserve que les formules A et B n'englobent pas les formules C et D 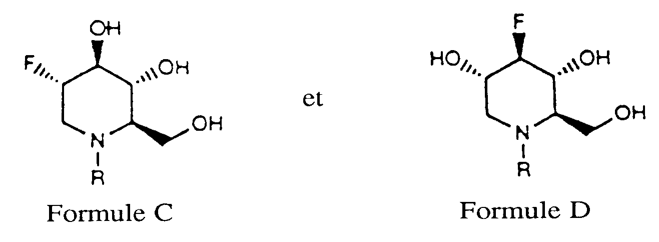 dans lesquelles les R représentent des atomes d'hydrogène, des groupes alkyle comportant de 1 à 10 atomes de carbone, des groupes alcényle comportant jusqu'à 10 atomes de carbone, des groupes aryle, alkaryle ou aralkyle comportant de 6 à 16 atomes de carbone, ou des groupes acyle ou acyloxy de formule 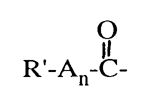 dans laquelle R' représente un groupe alkyle comportant de 1 à 10 atomes de carbone, ou un groupe aryle, alkaryle ou aralkyle comportant de 6 à 26 atomes de carbone, A représente un atome d'oxygène, et n vaut 0 ou 1.

2. Composés représentés par les formules 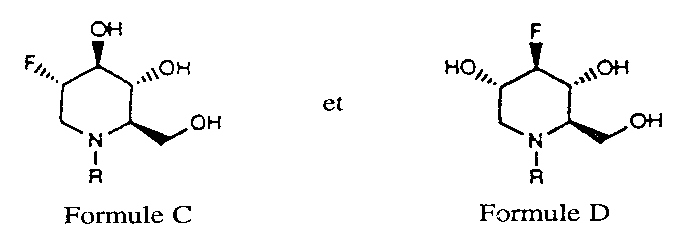 dans lesquelles les R représentent des atomes d'hydrogène, des groupes alkyle comportant de 1 à 10 atomes de carbone, des groupes alcényle comportant jusqu'à 10 atomes de carbone, des groupes aryle, alkaryle ou aralkyle comportant de 6 à 16 atomes de carbone, ou des groupes acyle ou acyloxy de formule 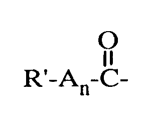 dans laquelle R' représente un groupe alkyle comportant de 1 à 10 atomes de carbone, ou un groupe aryle, alkaryle ou aralkyle comportant de 6 à 26 atomes de carbone, A représente un atome d'oxygène, et n vaut 0 ou 1.

3. Composés conformes à la revendication 2, dans lesquels R représente un atome d'hydrogène.

4. Composés conformes à la revendication 2, dans lesquels R représente un groupe alkyle comportant de 1 à 10 atomes de carbone.

5. Composés conformes à la revendication 2, dans lesquels R représente un groupe alkyle comportant de 1 à 6 atomes de carbone.

6. Composés conformes à la revendication 2, dans lesquels R représente un groupe alkyle comportant 4 atomes de carbone.

7. Composés conformes à la revendication 2, dans lesquels R représente un groupe n-butyle.

8. Composés conformes à la revendication 1, représentés par les formules 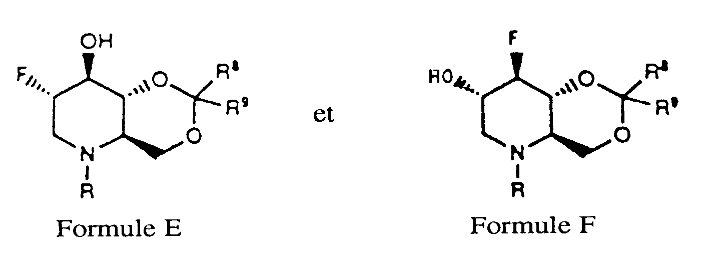 dans lesquelles
les R représentent des atomes d'hydrogène, des groupes alkyle comportant de 1 à 10 atomes de carbone, des groupes alcényle comportant jusqu'à 10 atomes de carbone, des groupes aryle, alkaryle ou aralkyle comportant de 6 à 16 atomes de carbone, ou des groupes acyle ou acyloxy définis dans la revendication 1; et
R⁸ et R⁹ représentent indépendamment des atomes d'hydrogène, des groupes alkyle comportant de 1 à 10 atomes de carbone, ou des groupes aryle.

9. Composés conformes à la revendication 8, dans lesquels R représente un atome d'hydrogène.

10. Composés conformes à la revendication 8, dans lesquels R représente un groupe alkyle comportant de 1 à 10 atomes de carbone.

11. Composés conformes à la revendication 8, dans lesquels R représente un groupe alkyle comportant de 1 à 6 atomes de carbone.

12. Composés conformes à la revendication 8, dans lesquels R représente un groupe alkyle comportant 4 atomes de carbone.

13. Composés conformes à la revendication 8, dans lesquels R représente un groupe n-butyle.

14. Composés conformes à la revendication 8, dans lesquels R représente un groupe phénoxycarbonyle.

15. Composés conformes à la revendication 8, dans lesquels R représente un groupe butyryle.

16. Composés conformes à la revendication 1, représentés par les formules 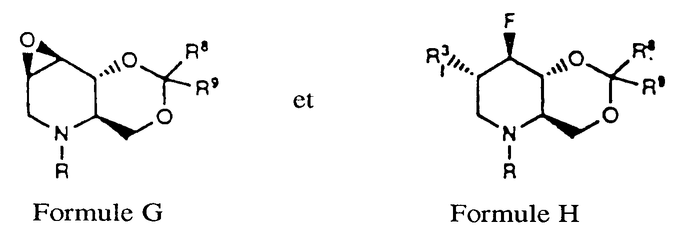 dans lesquelles
les R représentent des atomes d'hydrogène, des groupes alkyle comportant de 1 à 10 atomes de carbone, des groupes alcényle comportant jusqu'à 10 atomes de carbone, des groupes aryle, alkaryle ou aralkyle substitués, comportant de 7 à 14 atomes de carbone dans le cas de la formule G et de 6 à 16 atomes de carbone dans le cas de la formule H, ou des groupes acyle ou acyloxy définis dans la revendication 1;
R₁³ représente un groupe benzyloxy ou allyloxy, ou un groupe acyloxy représenté par la formule 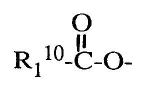 dans laquelle R₁¹⁰ représente un groupe alkyle comportant de 1 à 10 atomes de carbone, ou un groupe aryle, aralkyle ou alkaryle; et
R⁸ et R⁹ représentent indépendamment des atomes d'hydrogène, des groupes alkyle comportant de 1 à 10 atomes de carbone, ou des groupes aryle.

17. Composés conformes à la revendication 16, dans lesquels R représente un atome d'hydrogène.

18. Composés conformes à la revendication 16, dans lesquels R représente un groupe alkyle comportant de 1 à 10 atomes de carbone.

19. Composés conformes à la revendication 16, dans lesquels R représente un groupe alkyle comportant de 1 à 6 atomes de carbone.

20. Composés conformes à la revendication 16, dans lesquels R représente un groupe alkyle comportant 4 atomes de carbone.

21. Composés conformes à la revendication 16, dans lesquels R représente un groupe n-butyle.

22. Composés conformes à la revendication 16, dans lesquels R représente un groupe phénoxycarbonyle.

23. Composés conformes à la revendication 16, dans lesquels R représente un groupe butyryle.

24. Composés conformes à la revendication 1, représentés par les formules 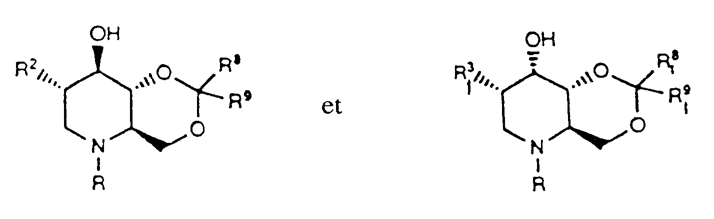 dans lesquelles
les R représentent des atomes d'hydrogène, des groupes alkyle comportant de 1 à 10 atomes de carbone, des groupes alcényle comportant jusqu'à 10 atomes de carbone, des groupes aryle, alkaryle ou aralkyle comportant de 6 à 16 atomes de carbone, ou des groupes acyle ou acyloxy définis dans la revendication 1;
R² représente un groupe ester sulfonique de formule 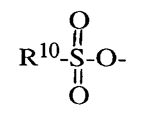 dans laquelle R¹⁰ représente un groupe alkyle comportant de 1 à 6 atomes de carbone, ou un groupe aryle, aralkyle ou alkaryle ;
R₁³ représente un groupe benzyloxy ou allyloxy, ou un groupe acyloxy représenté par la formule 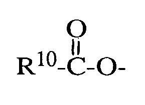 dans laquelle R¹⁰ représente un groupe alkyle comportant de 1 à 10 atomes de carbone, ou un groupe aryle, aralkyle ou alkaryle ; et
R⁸ et R⁹ représentent indépendamment des atomes d'hydrogène, des groupes alkyle comportant de 1 à 10 atomes de carbone, ou des groupes aryle.

25. Composés conformes à la revendication 24, dans lesquels R représente un atome d'hydrogène.

26. Composés conformes à la revendication 24, dans lesquels R représente un groupe alkyle comportant de 1 à 10 atomes de carbone.

27. Composés conformes à la revendication 24, dans lesquels R représente un groupe alkyle comportant de 1 à 6 atomes de carbone.

28. Composés conformes à la revendication 24 dans lesquels R représente un groupe alkyle comportant 4 atomes de carbone.

29. Composés conformes à la revendication 24, dans lesquels R représente un groupe n-butyle.

30. Composés conformes à la revendication 24, dans lesquels R représente un groupe phénoxycarbonyle.

31. Composés conformes à la revendication 24, dans lesquels R représente un groupe butyryle.

32. Composés conformes à la revendication 1, représentés par les formules 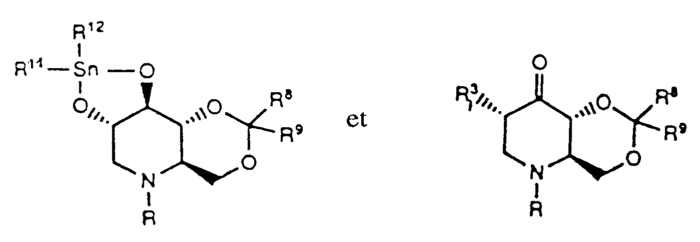 dans lesquelles
les R représentent des atomes d'hydrogène, des groupes alkyle comportant de 1 à 10 atomes de carbone, des groupes alcényle comportant jusqu'à 10 atomes de carbone, des groupes aryle, alkaryle ou aralkyle comportant de 6 à 16 atomes de carbone, ou des groupes acyle ou acyloxy définis dans la revendication 1;
R₁³ représente un groupe benzyloxy ou allyloxy, ou un groupe acyloxy représenté par la formule 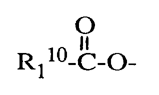 dans laquelle R₁¹⁰ représente un groupe alkyle comportant de 1 à 10 atomes de carbone, ou un groupe aryle, aralkyle ou alkaryle ;
R⁸ et R⁹ représentent indépendamment des atomes d'hydrogène, des groupes alkyle comportant de 1 à 10 atomes de carbone, ou des groupes aryle; et
R¹¹ et R¹² représentent des groupes alkyle comportant de 1 à 10 atomes de carbone.

33. Composés conformes à la revendication 32, dans lesquels R représente un atome d'hydrogène.

34. Composés conformes à la revendication 32, dans lesquels R représente un groupe alkyle comportant de 1 à 10 atomes de carbone.

35. Composés conformes à la revendication 32, dans lesquels R représente un groupe alkyle comportant de 1 à 6 atomes de carbone.

36. Composés conformes à la revendication 32, dans lesquels R représente un groupe alkyle comportant 4 atomes de carbone.

37. Composés conformes à la revendication 32, dans lesquels R représente un groupe n-butyle.

38. Composés conformes à la revendication 32, dans lesquels R représente un groupe phénoxycarbonyle.

39. Composés conformes à la revendication 32, dans lesquels R représente un groupe butyryle.

40. Composé conforme à la revendication 1, représenté par la formule 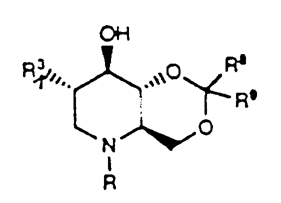 dans laquelle
R représente un atomes d'hydrogène, un groupe alkyle comportant de 1 à 10 atomes de carbone, un groupe alcényle comportant jusqu'à 10 atomes de carbone, un groupe aryle, alkaryle ou aralkyle comportant de 6 à 16 atomes de carbone, ou un groupe acyle ou acyloxy défini dans la revendication 1 ;
R₁³ représente un groupe benzyloxy ou allyloxy, ou un groupe acyloxy représenté par la formule 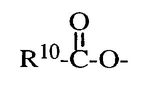 dans laquelle R¹⁰ représente un groupe alkyle comportant de 1 à 10 atomes de carbone, ou un groupe aryle, aralkyle ou alkaryle ; et
R⁸ et R⁹ représentent indépendamment des atomes d'hydrogène, des groupes alkyle comportant de 1 à 10 atomes de carbone, ou des groupes aryle.

41. Composé conforme à la revendication 40, dans lequel R représente un atome d'hydrogène.

42. Composé conforme à la revendication 40, dans lequel R représente un groupe alkyle comportant de 1 à 10 atomes de carbone.

43. Composé conforme à la revendication 40, dans lequel R représente un groupe alkyle comportant de 1 à 6 atomes de carbone.

44. Composé conforme à la revendication 40, dans lequel R représente un groupe alkyle comportant 4 atomes de carbone.

45. Composé conforme à la revendication 40, dans lequel R représente un groupe n-butyle.

46. Composé conforme à la revendication 40, dans lequel R représente un groupe phénoxycarbonyle.

47. Composé conforme à la revendication 40, dans lequel R représente un groupe butyryle.

48. Composition contenant un composé conforme à la revendication 2.

49. N-butyl-1,2-didésoxy-2-fluoro-nojirimycine.

50. 1,2-didésoxy-2-fluoro-nojirimycine.

51. N-butyl-1,3-didésoxy-3-fluoro-nojirimycine.

52. 1,3-didésoxy-3-fluoro-nojirimycine.
